# EUROPÄISCHE PATENTANMELDUNG

(11) **EP 3 730 037 A1**
(43) Veröffentlichungstag der Anmeldung: **28.10.2020**
(21) Anmeldenummer: 19170561.5
(22) Anmeldetag: 23.04.2019
(51) Int. Cl.: A61B 3/103, A61B 3/028

(54) **BESTIMMUNG EINES REFRAKTIONSFEHLERS EINES AUGES**

(71) Anmelder: Carl Zeiss Vision International GmbH, 73430 Aalen (DE)
(72) Erfinder: Leube, Alexander, 72072 Tübingen (DE); Ohlendorf, Arne, 72072 Tübingen (DE); Wahl, Siegfried, 73072 Donzdorf (DE)
(74) Vertreter: Altmann Stößel Dick Patentanwälte PartG mbB

(57) **Zusammenfassung**

Die vorliegende Erfindung betrifft ein Verfahren (210), eine Vorrichtung (110) und ein Computerprogramm zur Bestimmung eines Refraktionsfehlers eines Auges (112) eines Nutzers (114), sowie ein Verfahren zur Herstellung eines Brillenglases für das Auge (112) des Nutzers (114). Das Verfahren (210) zur Bestimmung eines Refraktionsfehlers eines Auges (112) eines Nutzers (114) umfasst hierbei die folgenden Schritte:
a) Darstellen eines Zeichens (122) auf einem Bildschirm (120), wobei ein Parameter des auf dem Bildschirm (120) dargestellten Zeichens (122) verändert wird;
b) Erfassen einer Augenbewegungsmetrik (144) des Auges (112) des Nutzers (114) in Abhängigkeit von dem auf dem Bildschirm (120) dargestellten Zeichen (122);
c) Feststellen eines Zeitpunkts, zu dem sich aus der Augenbewegungsmetrik (144) des Auges (112) des Nutzers (114) eine Erkennungsschwelle des Nutzers (114) für das auf dem Bildschirm (120) dargestellten Zeichens (122) ergibt; und
d) Bestimmen eines Wertes (220) für den Refraktionsfehler des Auges (112) des Nutzers (114) aus dem zu dem Zeitpunkt festgelegten Parameter.

Die vorliegenden Erfindung ermöglicht eine objektive Bestimmung des Wertes (220) für den Refraktionsfehler des Auges (112) des Nutzers (114) ohne Einsatz von Spezialgeräten, ohne Feedback durch den Nutzer (114), ist nicht an einen Ort gebunden und ist auch von Laien benutzbar.

## Beschreibung

### Gebiet der Erfindung

Die vorliegende Erfindung betrifft ein Verfahren, eine Vorrichtung und ein Computerprogramm zur Bestimmung eines Refraktionsfehlers eines Auges eines Nutzers, sowie ein Verfahren zur Herstellung eines Brillenglases für das Auge des Nutzers.

### Stand der Technik

Aus dem Stand der Technik sind Verfahren zur Bestimmung von Refraktionsfehlern eines Auges eines Nutzers bekannt. Der Begriff "Refraktion" bezeichnet hierbei eine Lichtbrechung im Auge des Nutzers, die ein durch die Pupille in das Innere des Auges einfallender Lichtstrahl erfährt. Die Defokussierung des Auges des Nutzers, also ein spezifischer Refraktionsfehler, kann zu einer Fehlsichtigkeit (Ametropie) des Nutzers führen, insbesondere zu einer Kurzsichtigkeit (Myopie) oder zu einer Weitsichtigkeit (Hyperopie) des Nutzers. Zur subjektiven Bestimmung der Refraktion werden üblicherweise Sehzeichen, bevorzugt in Form von Zahlen, Buchstaben oder Symbolen, auf einer Tafel oder einem Bildschirm in einer festgelegten Größe für eine gegebene Entfernung bereitgestellt, die der Nutzer betrachtet. Durch ein Vorhalten einer Anzahl von optischen Linsen mit bekannten Eigenschaften sowie durch eine Führung des Nutzers in einem Frageprozess kann subjektiv bestimmt werden, welche Defokussierung das Auge des Nutzers aufweist und welche sphärozylindrische Ausgestaltung des Brillenglases zu einem weitgehenden Ausgleich der Refraktionsfehler des Auges und damit zu einer möglichst optimalen Bildqualität für den Nutzer führt.

Weiterhin ist es bei bekannten Verfahren zur objektiven Bestimmung der Refraktion des Auges erforderlich, dass das hierzu eingerichtete optische Messgerät sowie die hierzu erforderliche Lichtquelle mit der optischen Achse des Auges ausgerichtet werden. Daher können bekannte Vorrichtungen zur objektiven Bestimmung der Refraktion meist nur von Fachpersonal, insbesondere von einem Augenoptiker, bedient werden und sind in der Regel ortsfest und daher nicht mobil einsetzbar.

US 2014/268060 A1 offenbart eine Vorrichtung und ein Verfahren zur Bestimmung der Refraktion eines Auges und eines Astigmatismus unter Verwendung eines Computer-Bildschirms. Hierzu wird ein Sehzeichen auf dem Bildschirm dargestellt und durch Veränderung der auf dem Bildschirm dargestellten Größe des Sehzeichens ein Wert für die Größe des Sehzeichens ermittelt, bei welchem das Sehzeichen für den Nutzer gerade nicht mehr erkennbar ist.

WO 2018/077690 A1 offenbart Vorrichtungen und ein Computerprogramm, mit welchem die sphärozylindrische Refraktion eines Auges bestimmt werden kann. Hierzu wird eine Komponente mit einer einstellbaren Optik bereitgestellt, welche über eine BrechkraftEinstelleinrichtung hinsichtlich ihrer Brechkraft verstellt werden kann. Aus der Einstellung der Brechkrafteinstelleinrichtung bei verschiedenen Orientierungen einer Vorzugsrichtung der Optik oder einer Vorzugsrichtung von Sehzeichen wird dann die sphärozylindrische Refraktion bestimmt.

Weiterhin sind Verfahren und Vorrichtungen zur Bestimmung von Augenbewegungen eines Nutzers bekannt.

US 6,402,320 B1 offenbart ein automatisches Verfahren zur Bestimmung einer visuellen Sehschärfe, insbesondere für Kleinkinder, mittels einer elektronischen visuellen Anzeigeeinrichtung, das folgende Verfahrensschritte umfasst: (a) Bereitstellen eines Fixationsziels auf der Anzeigeeinrichtung als Stimulus für den Nutzer; dann (b) Bereitstellen eines Testbildes auf der Anzeigeeinrichtung, wobei das Testbild mindestens zwei separate Felder umfasst, wobei eines der Felder ein erstes Testmuster und ein anderes der Felder ein Kontrollmuster aufweist, wobei das Testmuster als Stimulus für den Nutzer eingerichtet ist, wenn das Testmuster für den Nutzer erkennbar ist; dann (c) Nachweisen, ob eine Augenbewegung zu dem Testmuster auftritt, wobei ein Auftreten einer Augenbewegung zu dem Testmuster die Unterscheidbarkeit des ersten Testmusters durch den Nutzer bestätigt; dann (d) Wiederholen der Schritte (b) und (c) mit einem weiteren Testmuster, wobei das weitere Testmuster schwerer unterscheidbar ist als das erste Testmuster; und (e) Bestimmen der visuellen Sehschärfe des Nutzers aus dem Auftreten oder Abwesenheit der Augenbewegung zu dem ersten Testmuster und zu wenigstens einem weiteren Testmuster.

Rucci, M., Iovin, R, Poletti, M., & Santini, F. (2007), Miniature eye movements enhance fine spatial detail, Nature, 447 (7146), 852 berichten über eine Analyse des Einflusses von fixierenden Augenbewegungen auf eine Unterscheidung von Gittern, die mittels Rauschen, das ein ähnliches Leistungsspektrum wie natürliche Bilder aufweist, maskiert wurden. Mittels eines Verfahrens zur Stabilisierung des Netzhautbildes konnte die Bewegung des Netzhautbildes, die zwischen benachbarten Sprüngen von Augenbewegungen (so genannten Sakkaden) zur Erfassung eines neuen Fixationspunktes auftritt, selektiv eliminiert werden. In dieser Veröffentlichung wird gezeigt, dass fixierende Augenbewegungen die Unterscheidung von hochfrequenten räumlichen Stimuli verbessert, jedoch nicht von niederfrequenten räumlichen Stimuli. Diese Verbesserung stammt aus zeitlichen Modulationen, die durch die fixierenden Augenbewegungen in den visuellen Eingang der Netzhaut eingeführt werden, welche die hochfrequenten räumlichen Stimuli betonen. Bei natürlichem Sehen, das von niederfrequenten räumlichen Stimuli dominiert wird, scheinen die fixierenden Augenbewegungen eine effektive Strategie zur Verarbeitung von räumlichen Details darzustellen.

US 2015/070273 A1 offenbart Verfahren und Vorrichtungen zum optischen Nachweis und Verfolgen einer Augenbewegung. Ein Verfahren zur Verfolgung der Augenbewegung umfasst Emittieren von Licht auf das Auge des Nutzers unter Verwendung von mehreren, im Wesentlichen gleichweit von einem Photodetektor-Modul der Vorrichtung entfernten Lichtquellen, Empfangen Modul einer wenigstens teilweisen Rückstrahlung des von jeder der mehreren Lichtquellen emittierten und von dem Auge zurückgestrahlten Lichts in dem Photodetektor, und Bestimmen eines Positionsparameters des Auges basierend auf verschiedenen Werten der wenigstens teilweisen ückstrahlung des Lichts in Bezug auf die mehreren Lichtquellen.

Denniss, J., Scholes, C., McGraw, P. V., Nam, S. H., & Roach, N. W. (2018). Estimation of Contrast Sensitivity From Fixational Eye Movements. Investigative Ophthalmology & Visual Science, 59 (13), S. 5408-16, beschreiben ein Verfahren zur objektiven und verlässlichen Abschätzung von Kontrastempfindlichkeit aus der Beobachtung von Mikrosakkaden. Hieraus erhaltene Ergebnisse deuten darauf hin, dass die Rate von Mikrosakkaden mit zunehmendem Kontrast in einem Bild ebenfalls zunimmt.

### Aufgabe der Erfindung

Insbesondere ausgehend von der Offenbarung der US 6,402,320 B1 besteht die Aufgabe der vorliegenden Erfindung darin, ein Verfahren, eine Vorrichtung und ein Computerprogramm zur Bestimmung eines Refraktionsfehlers eines Auges eines Nutzers, sowie ein Verfahren zur Herstellung eines Brillenglases für das Auge des Nutzers bereitzustellen, welche die aufgeführten Nachteile und Einschränkungen des Standes der Technik zumindest teilweise überwinden.

Insbesondere sollen das Verfahren, die Vorrichtung und das Computerprogramm die Bestimmung des Refraktionsfehlers des Auges ermöglichen, ohne dass auf eine subjektive Reaktion des Nutzers zurückgegriffen werden muss. Die Bestimmung des Refraktionsfehlers des Auges des Nutzers soll hierbei ohne Spezialgeräte erfolgen und daher auch von Laien ausgeführt werden können.

### Offenbarung der Erfindung

Diese Aufgabe wird gelöst durch ein Verfahren, eine Vorrichtung und ein Computerprogramm zur Bestimmung eines Refraktionsfehlers eines Auges eines Nutzers, sowie ein Verfahren zur Herstellung eines Brillenglases für das Auge des Nutzers mit den Merkmalen der unabhängigen Patentansprüche. Bevorzugte Ausgestaltungen, welche einzeln oder in Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben dem durch diese Begriffe eingeführten Merkmal, keine weiteren Merkmale vorhanden sind oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist, d.h. auf eine Situation, in welcher A ausschließlich aus B besteht, als auch auf die Situation, in welcher, zusätzlich zu B, ein oder mehrere weitere Elemente in A vorhanden sind, beispielsweise Element C, Elemente C und D oder sogar weitere Elemente.

In einem ersten Aspekt betrifft die vorliegende Erfindung ein Verfahren zur Bestimmung eines Refraktionsfehlers eines Auges eines Nutzers. Hierbei kann sich das Verfahren auf die Bestimmung des Refraktionsfehlers eines Auges oder beider Augen eines Nutzers beziehen. Das Verfahren umfasst die folgenden Schritte a) bis d), vorzugsweise in der angegebenen Reihenfolge. Auch eine andere Reihenfolge ist grundsätzlich möglich. Insbesondere ist auch eine ganz oder teilweise zeitgleiche Ausführung der Schritte möglich. Weiterhin können einzelne, mehrere oder alle Schritte des Verfahrens wiederholt, insbesondere mehr als einmal, ausgeführt werden. Das Verfahren kann, zusätzlich zu den genannten Schritten auch weitere Verfahrensschritte umfassen.

Das Verfahren zur Bestimmung eines Refraktionsfehlers eines Auges eines Nutzers umfasst die Schritte:
a) Darstellen eines Zeichens auf einem Bildschirm, wobei ein Parameter des auf dem Bildschirm dargestellten Zeichens verändert wird;
b) Erfassen einer Augenbewegungsmetrik des Auges des Nutzers in Abhängigkeit von dem auf dem Bildschirm dargestellten Zeichen;
c) Feststellen eines Zeitpunkts, zu dem sich aus der Augenbewegungsmetrik des Auges des Nutzers eine Erkennungsschwelle des Nutzers für das auf dem Bildschirm dargestellte Zeichen ergibt; und
d) Bestimmen eines Wertes für den Refraktionsfehler des Auges des Nutzers aus dem zu dem Zeitpunkt festgelegten Parameter.

Das hier vorgeschlagene Verfahren zur Bestimmung eines Refraktionsfehlers eines Auges eines Nutzers eignet sich insbesondere zur Verwendung in einem Verfahren zur Herstellung eines Brillenglases für das Auge des betreffenden Nutzers. Unter einem "Brillenglas" wird gemäß Norm DIN EN ISO 13666:2013-10, im Folgenden auch als die "Norm" bezeichnet, Abschnitte 8.1.1 und 8.1.2 eine optische Linse verstanden, welche im Rahmen der vorliegenden Erfindung zur Korrektion von Fehlsichtigkeiten des Auges dienen soll, wobei die optische Linse vor dem Auge des Nutzers, aber nicht in Kontakt mit dem Auge getragen wird.

Der Begriff der "Brille" bezeichnet im Rahmen der vorliegenden Erfindung ein beliebiges Element, welches zwei einzelne Brillengläser und eine Brillenfassung umfasst, wobei das Brillenglas zur Einbringung in eine Brillenfassung vorgesehen ist, welche von einem Nutzer der Brille ausgewählt wird. Anstelle des hier verwendeten Begriffs des "Nutzers" kann gleichbedeutend auch einer der Begriffe "Subjekt", "Brillenträger", "Benutzer" oder "Proband" verwendet werden.

Gemäß Schritt a) des vorliegenden Verfahrens erfolgt eine Darstellung eines Zeichens auf einem Bildschirm, wobei ein Parameter des auf dem Bildschirm dargestellten Zeichens verändert wird. Der Begriff "Bildschirm" bezeichnet hierbei eine elektronisch ansteuerbare Anzeige, welche über eine zwei-dimensionale Ausdehnung verfügt, wobei das gewünschte Zeichen mit weitgehend frei wählbaren Parametern an einer beliebigen Stelle innerhalb der Ausdehnung darstellbar ist. Der Bildschirm kann hierbei bevorzugt ausgewählt sein aus einem Monitor, einem Screen oder einem Display. Vorzugsweise kann der Bildschirm hierbei von einen mobilen Kommunikationsgerät umfasst sein. Der Begriff des "mobilen Kommunikationsgeräts" umfasst hierbei insbesondere ein Mobiltelefon (Handy), ein Smartphone oder ein Tablet. Andere Arten von mobilen Kommunikationsgeräten sind jedoch denkbar. Auf diese Weise kann das vorliegende Verfahren zur Bestimmung eines Refraktionsfehlers des Auges an einem beliebigen Ort durchgeführt werden. Andere Arten von Bildschirmen sind jedoch ebenfalls möglich.

Der Begriff des "Zeichens" betrifft hierbei Sehzeichen, insbesondere Buchstaben, Zahlen oder Symbole; Bilder oder Muster, welche farbig oder in schwarz-weiß dargestellt werden können. Während es sich bei den "Sehzeichen" jeweils um ein einzelnes feststehendes Zeichen handelt, das in seinem Proportionen zur Wiedererkennbarkeit durch den Nutzer nur eingeschränkt veränderbar ist, bezeichnet der Begriff des "Musters" eine beliebige graphische Struktur, welche - insbesondere im Gegensatz zu einem Rauschen, das ohne erkennbare Struktur bleibt - über mindestens eine räumlich orientierte Periode verfügt, innerhalb der die Struktur des Musters wiederholt dargestellt ist. Anstelle des Begriffs des "Musters" wird daher auch der Begriff des "periodischen Musters" verwendet, um diese Eigenschaft des Musters deutlich zum Ausdruck zu bringen. Im Folgenden sollen diese beiden Begriffe jedoch denselben Begriffsinhalt umfassen.

In einer bevorzugten Ausgestaltung kann das Zeichen derart auf der Ausdehnung des Bildschirms dargestellt werden, dass eine Richtung in Bezug auf das Zeichen, beispielsweise eine Kante des Zeichens, einen festgelegten Winkel in Bezug auf eine Orientierung des Bildschirms einnehmen, wobei der festgelegte Winkel bevorzugt 0° oder ein Vielfaches von 90° ist. Der Begriff "Orientierung" bezeichnet hierbei eine Richtung, welche parallel zu einem Rand des Bildschirms ist, der üblicherweise die Form eines Rechtecks annimmt. Auf diese Weise kann das Zeichen an die vorhandene Ausdehnung des Bildschirms angepasst sein. Andere Arten der Darstellung des Zeichens auf dem Bildschirm sind jedoch denkbar, beispielsweise mit einem in Bezug auf die Orientierung des Bildschirms festgelegten Winkel von 45° oder einem ungeraden Vielfachen davon.

Aufgrund einer elektronischen Ansteuerung kann ein Parameter des auf dem Bildschirm dargestellten Zeichens auf einfache Weise und in einem weiten Rahmen verändert werden. Bei dem "Parameter" handelt es sich um eine Eigenschaft des Zeichens, je nach ausgewähltem Zeichen, insbesondere um eine Ausdehnung, eine Orientierung, eine Frequenz, einen Kontrast oder eine Farbe (einschließlich schwarz und weiß). Im Falle des Musters kann eine Struktur wiederholt dargestellt werden, wobei sich durch Wiederholung gleichartige Punkte oder Bereiche über die Struktur des Musters ausbilden können. Bevorzugte Ausgestaltungen gleichartiger Punkte oder Bereiche können bevorzugt als periodische Maxima oder Minima des Musters vorliegen. Während sich es bei den ausgewählten Parametern eines herkömmlichen Sehzeichens, insbesondere einem Buchstaben, einer Zahl oder einem Symbol, also um eine Ausdehnung, insbesondere eine Höhe oder Breite, des Zeichens handeln kann, bezieht sich bei dem periodischen Muster der Parameter bevorzugt auf einen Parameter einer periodischen Funktion, insbesondere eine Wiederholfrequenz. Die "periodische Funktion" bezeichnet hierbei eine Anweisung an eine Ausgestaltung einer zeitlich oder bevorzugt räumlich wiederholten Veränderung des Musters. Die periodische Funktion kann vorzugsweise ausgewählt sein aus einer Sinusfunktion, einer Cosinusfunktion oder einer Überlagerung hiervon. Andere periodische Funktionen sind jedoch denkbar.

In einer besonders bevorzugten Ausgestaltung der vorliegenden Erfindung kann das auf dem Bildschirm dargestellte Zeichen eine Bewegung über den Bildschirm ausführen. Die Bewegung des auf dem Bildschirm dargestellten Zeichens kann hierbei kontinuierlich oder in Sprüngen erfolgen. Der Begriff der "Bewegung" bezeichnet hierbei eine Veränderung des Ortes auf dem Bildschirm, auf dem das Zeichen dargestellt ist, mit der Zeit, unabhängig davon, ob sich das auf dem Bildschirm dargestellte Zeichen im Hinblick auf seine Parametrisierung während eines Ablaufs der Bewegung ändert oder nicht. Erfindungsgemäß ist es jedoch besonders bevorzugt, wenn der Parameter des auf dem Bildschirm dargestellten Zeichens verändert wird, während das auf dem Bildschirm dargestellte Zeichen eine Bewegung über den Bildschirm ausführt. Hierbei ist unerheblich, dass die Bewegung des Zeichens über den Bildschirm nur scheinbar erfolgt. Aufgrund der elektronischen Ansteuerung kann der Ort der Darstellung des Zeichens auf dem Bildschirm derart auf einfache Weise verändert werden, dass die Veränderung schneller, als vom Nutzer erkennbar, erfolgen kann, so dass der Nutzer annehmen kann, dass das Zeichen tatsächlich eine Bewegung über den Bildschirm ausführe. Der Nutzer kann hierbei in der Regel eine kontinuierliche Bewegung erkennen, wenn sich der Ort der Darstellung des Zeichens auf dem Bildschirm entsprechend langsam verändert. Andererseits kann eine schnelle oder große räumliche Veränderung des Ortes der Darstellung des Zeichens auf dem Bildschirm den Nutzer annehmen lassen, dass das Zeichen über den Bildschirm springe.

In einer bevorzugten Ausgestaltung kann das auf dem Bildschirm dargestellte Zeichen ein Muster sein, wobei der zugehörige Parameter des Musters mindestens eine Ortsfrequenz des periodischen Musters umfassen. Der Begriff der "Ortsfrequenz" bezeichnet hierbei einen Kehrwert eines räumlichen Abstands, welcher in der Einheit 1/m oder insbesondere bei Kenntnis einer Entfernung des Bildschirms von dem Auge des Nutzers alternativ oder zusätzlich auch als dimensionslose Zahl, zum Beispiel pro Grad oder pro Zyklus, angegeben werden kann, zwischen zwei benachbart angeordneten gleichartigen Punkten, insbesondere einem Maximum oder einem Minimum, in einer räumlich periodischen Änderung des Musters. Bevorzugt kann hierbei die Intensität oder die Farbe des Musters entlang einer Richtung der Ausdehnung des Bildschirms dem Verlauf der periodischen Funktion, insbesondere der Sinusfunktion, folgen. Andere Arten der Bestimmung der Ortsfrequenz aus dem Muster sind jedoch denkbar, beispielsweise aus einem Abstand von Punkten gleicher Intensität.

In dieser bevorzugten Ausgestaltung kann das periodische Muster als zweidimensionale Überlagerung einer periodischen Funktion, insbesondere der Sinusfunktion, welche sich in eine erste Richtung auf der Ausdehnung des Bildschirms erstrecken kann, und einer konstanten Funktion, welche sich in eine zweite Richtung auf der Ausdehnung des Bildschirms erstreckten kann, die vorzugsweise senkrecht zur ersten Richtung angeordnet sein kann, ausgestaltet sein. Der Begriff "senkrecht" bezeichnet hierbei einen Winkel von 90° ± 30°, bevorzugt von 90° ± 15°, besonders bevorzugt von 90° ± 5°, insbesondere von 90° ± 1°. Andere Winkel zwischen der ersten Richtung und der zweiten Richtung sind jedoch ebenfalls möglich. Auf diese Weise kann das Muster in Form von periodisch nebeneinander angeordneten Streifen vorliegen, die auch als "sinusförmiges Gitter" oder "Gabor Patch" bezeichnet werden können. Der Begriff "Gabor Patches" bezeichnet sinusförmige Gitter, die üblicherweise mit einer Gauß-förmigen Hülle versehen sind, und die dafür bekannt sind, dass sie sich insbesondere als Stimulus für das Auge des Nutzers verwenden lassen. Andere Arten von Mustern sind jedoch möglich.

Gemäß Schritt b) erfolgt in Abhängigkeit von dem auf dem Bildschirm dargestellten Zeichen, das als Stimulus für das Auge des Nutzers dient, eine Erfassung einer Augenbewegungsmetrik des Auges des Nutzers, vorzugsweise während der Darstellung des Zeichens auf dem Bildschirm gemäß Schritt a). Der Begriff "Augenbewegungsmetrik" bezeichnet hierbei ein Maß, welches mit einer Bewegung des Auges des Nutzers verknüpft ist, wobei die Bewegung des Auges des Nutzers von einem äußeren, auf das Auge des Nutzers einwirkenden Stimulus hervorgerufen wird. Im Rahmen der vorliegenden Erfindung kann sich die Augenbewegungsmetrik vorzugsweise beziehen auf: eine Augenfolgebewegung; eine Augenbewegung, die sich auf Mikrosakkaden bezieht, die eine Mikrosakkadenrichtung, eine Mikrosakkadenrate oder eine Sakkadengenauigkeit umfasst; oder einen optokinetischen Nystagmus. Weitere Augenbewegungsmetriken können zum Beispiel die Verweildauer beim flüssigen Lesen des dargestellten Zeichens, die auch als "Fixationsdauer" bezeichnet wird, umfassen. Weitere Arten an Augenbewegungen können darüber hinaus ebenfalls erfasst werden. Welche Art von Augenbewegungsmetrik oder welche Kombination von mindestens zwei Augenbewegungsmetriken zur Bestimmung des Wertes für den Refraktionsfehler des Auges des Nutzers, insbesondere zur unten näher erläuterten Ermittlung der Kontrastempfindlichkeit bei den Ortsfrequenzen des Musters herangezogen werden, hängt im Wesentlichen von einer Genauigkeit einer hierzu verwendeten Videokamera ab.

Der Begriff "Augenfolgebewegung" bezeichnet hierbei eine Bewegung des Auges, durch welche das Auge den Bewegungen des auf dem Bildschirm dargestellten Zeichens, das von dem Auge fixiert wird, nachfolgt. Im Allgemeinen handelt es sich bei der Augenfolgebewegung um eine langsame Bewegung des Auges mit einer Winkelgeschwindigkeit von 0,5 °/s bis 50 °/s, bei welcher ein Abbild des Zeichens vorzugsweise auf der Fovea des Auges verbleibt. Die Augenfolgebewegungen können nicht willkürlich erzeugt werden, sondern setzen voraus, dass das auf dem Bildschirm dargestellte Zeichen eine Bewegung ausführt, welcher das Auge des Nutzers folgen kann.

Augenbewegungsmetriken, die sich auf Sakkaden oder Mikrosakkaden beziehen, können im Rahmen der vorliegenden Erfindung vorzugsweise als Maß dafür verwendet werden, um festzustellen, ob der Nutzer das auf dem Bildschirm dargestellte Zeichen als Stimulus erkannt hat oder nicht. Der Begriff "Sakkade" bezeichnet ruckartige Blickzielbewegungen des Auges des Nutzers, die zielbezogen ausgeführt werden, die eine geringe Amplitude von mindestens 1° aufweisen und die insbesondere zu einer schnellen regelmäßigen Neuausrichtung einer Blickrichtung des Auges auf einen Fixationspunkt dienen, vorzugsweise indem das Abbild eines sich in dem Fixationspunkt befindlichen Zeichens aus einer Peripherie auf die Fovea des Auges verschoben wird. Eine "Sakkadenrate" beträgt typischerweise 1 Hz bis 5 Hz, wobei eine Winkelgeschwindigkeit von 5 °/s bis 500 °/s erreicht werden kann. Der Begriff "Mikrosakkade" bezeichnet kleine ruckartige und unwillkürliche Blickbewegungen, die nicht auf ein Ziel bezogen sein können, die zufällig auftreten und deren Amplitude geringer als 1° ist. Die "Mikrosakkadenrichtung" betrifft eine räumliche Orientierung der Mikrosakkade relativ zu einem Koordinatensystem, vorzugsweise einem durch den Bildschirm festgelegten Koordinatensystem. Hierbei kann die Orientierung relativ zu dem auf dem Bildschirm dargestellten Zeichen als Maß des Erkennens dienen. Die "Sakkadengenauigkeit" bezeichnet eine räumliche Präzision einer Neuausrichtung relativ zu einer neuen Position eines Stimulus. Kann der Stimulus nach der Neuausrichtung schlechter wahrgenommen werden, ist hierbei ein zu erwartender Fehler der Neuausrichtung größer.

Alternativ oder zusätzlich können Augenbewegungsmetriken, welche sich auf den optokinetischen Nystagmus beziehen, vorzugsweise als Maß dafür verwendet werden, um festzustellen, ob der Nutzer das auf dem Bildschirm dargestellte Zeichen als Stimulus erkannt hat oder nicht. Der Begriff "optokinetischer Nystagmus" bezeichnet einen physiologischen Augenbewegungsreflex, welcher durch eine langsame und eine schnelle Phase charakterisiert ist. Hierbei entspricht die langsame Phase einer Folgebewegung der Geschwindigkeit eines bewegten Stimulus' der Umgebung. Eine Korrelation der Phase oder der Geschwindigkeit des Stimulus' auf dem Bildschirm mit der langsamen Phase des optokinetischen Nystagmus kann als Maß dafür verwendet werden, ob ein Nutzer den Stimulus erkennt.

Zur Erfassung der Augenbewegungsmetriken kann hierbei vorzugsweise eine Videokamera eingesetzt werden, insbesondere um ein videobasiertes "Eyetracking" durchführen zu können. Der Begriff der "Videokamera" bezeichnet hierbei eine Kamera, die dazu eingerichtet ist, Bildfolgen einer Augenpartie des Nutzers aufnehmen. Aus den von der Videokamera aufgenommenen Bildfolgen können mittels Bildverarbeitung, vorzugsweise in einer dafür eingerichteten Auswerteeinheit, die mindestens eine beobachtete Augenbewegungsmetrik ausgewertet werden. Hierfür lassen sich insbesondere jeweils bekannte Algorithmen einsetzen.

Darüber hinaus können aus den von der Videokamera aufgenommenen Bildfolgen mittels Bildverarbeitung, ebenfalls bevorzugt in der Auswerteeinheit, weiterhin geometrische Daten des Auges, bevorzugt der Pupille, insbesondere Position und Durchmesser der Pupille, ermittelt werden und hieraus zum Beispiel die Blickrichtung des Auges bestimmt werden. Weiterhin sind Verfahren einsetzbar, welche ausgewählte Reflexionspunkte einbeziehen, die auf Vorderseite und/oder Rückseite von Cornea und Linse entstehen können, wenn das Auge durch eine Lichtquelle angestrahlt wird. Insbesondere kann ein Hornhautreflex bestimmt werden, siehe zum Beispiel P. Blignaut, Mapping the Pupil-Glint Vector to Gaze Coordinates in a Simple Video-Based Eye Tracker, Journal of Eye Movement Research 7(1):4, Seite 1-11, 1995. Grundsätzlich können aber auch andere Reflexe aufgenommen werden, insbesondere mittels eines so genannten "Dual Purkinje Eyetrackers". Da sich der Hornhautreflex ohne Kopfbewegung nicht bewegt, jedoch die Pupille ihre Position bei der Augenbewegung ändert, kann hieraus auf die Rotation des Auges geschlossen werden. Die "Pupille" bezeichnet hierbei eine in jedem Auge vorhandene Eintrittsöffnung, durch welche Strahlung in Form von Licht in das Innere des Auges eintreten kann. In umgekehrter Richtung kann die Pupille als Austrittsöffnung betrachtet werden, durch welche die Blickrichtung des Nutzers aus dem Auge auf die Umgebung festgelegt werden kann.

Die Videokamera kann vorzugsweise von demselben mobilen Kommunikationsgerät wie der Bildschirm umfasst sein. Insbesondere kann es sich hierbei um eine Rückkamera oder bevorzugt um eine Frontkamera des mobilen Kommunikationsgeräts handeln. Auf diese Weise kann die gewünschte Bildfolge der Augenpartie des Nutzers mittels der Kamera in vorteilhafter Weise an jedem beliebigen Ort aufgenommen werden. Andere Arten von Kameras sind jedoch denkbar, beispielsweise eine Kamera, die als brillenähnliches Gerät tragbar ist und vom Bildschirm getrennt ist. Nachteilig hieran ist, dass Display und Kamera synchronisiert werden müssen.

Weiterhin kann eine Beleuchtungseinrichtung vorgesehen sein, insbesondere um die Augenbewegungsmetrik des Nutzers mit möglichst hoher Auflösung und hohem Kontrast mittels der Videokamera erfassen zu können. Alternativ oder zusätzlich kann auf Tageslicht oder auf eine bereits vorhandene Beleuchtung zurückgegriffen werden.

In einer besonderen Ausgestaltung kann die die Videokamera eine Sensitivität im infraroten Spektralbereich, d.h. bei einer Wellenlänge von 780 nm bis 1 mm, bevorzugt von 780 nm bis 3 µm, insbesondere von 780 nm bis 1,4 µm (nach der Norm, Abschnitt 4.4, auch als "IR-A" bezeichnet), aufweisen. Um eine Infrarotstrahlung bereitzustellen, kann hierfür eine Lichtquelle vorgesehen sein, welche im infraroten Spektralbereich abstrahlt, insbesondere mit einer Wellenlänge, für welche die Videokamera eine ausreichende Sensitivität aufweist. Die Lichtquelle kann vorzugsweise ausgewählt sein aus einer Mikroglühlampe, einem IR-Emitter auf Festkörperbasis, einer lichtemittierenden Diode oder einem Infrarotlaser, wobei entsprechende Filter verwendet werden können.

Gemäß Schritt c) erfolgt, vorzugsweise während der Ausführung von Schritt b), eine Feststellung eines Zeitpunkts, der dadurch festgelegt ist, dass sich zu dem festgestellten Zeitpunkt aus der Beobachtung der Augenbewegungsmetrik des Nutzers eine Erkennungsschwelle des Nutzers für das auf dem Bildschirm dargestellte Zeichen ergibt. Hierzu kann Schritt b) für verschiedene Werte des Parameters wiederholt werden, vorzugsweise solange bis der gewünschte Zeitpunkt durch die Ausführung von Schritt c) festgestellt wurde. Hierbei bezeichnet der Begriff "Erkennungsschwelle", dass der Nutzer das auf dem Bildschirm dargestellte Zeichen als Stimulus für das Auge gerade noch oder gerade erst wahrnehmen kann. Nimmt einer der Parameter des Zeichens, insbesondere die Ortsfrequenz in dem periodischen Muster, zunehmend zu, kann hierbei der Zeitpunkt festgestellt werden, an dem das auf dem Bildschirm dargestellte Zeichen gerade nicht mehr als Stimulus für das Auge des Nutzers wirken kann. Umgekehrt kann, falls einer der Parameter des Zeichens, insbesondere die Ortsfrequenz in dem periodischen Muster, zunehmend abnimmt, hierbei der Zeitpunkt festgestellt werden, an dem das auf dem Bildschirm dargestellte Zeichen erstmals nicht als Stimulus für das Auge des Nutzers wirken kann. Alternativ kann auch, beispielsweise, wenn einer der Parameter des Zeichens, insbesondere die Ortsfrequenz in dem periodischen Muster, zunehmend abnimmt, hierbei der Zeitpunkt festgestellt werden, an dem das auf dem Bildschirm dargestellte Zeichen gerade als Stimulus für das Auge des Nutzers wirken kann. Umgekehrt kann in diesem Fall dann, falls einer der Parameter des Zeichens, insbesondere die Ortsfrequenz in dem periodischen Muster, zunehmend zunimmt, hierbei der Zeitpunkt festgestellt werden, an dem das auf dem Bildschirm dargestellte Zeichen erstmals als Stimulus für das Auge des Nutzers wirken kann.

In einer besonders bevorzugten Ausgestaltung der vorliegenden Erfindung kann der Zeitpunkt, zu dem sich aus der Reaktion des Nutzers die Erkennungsschwelle des Nutzers für das auf dem Bildschirm dargestellte Zeichen ergibt, dadurch festgestellt werden, dass die Augenbewegungsmetrik des Nutzers der Bewegung des auf dem Bildschirm dargestellten Zeichens gerade noch folgt oder gerade erst beginnt zu folgen. Insbesondere kann hierzu die Augenfolgebewegung des Nutzers, mit welcher er den Bewegungen eines Zeichens, das von dem Auge fixiert wird, nachfolgt, zur Feststellung des gewünschten Zeitpunkts verwendet werden, insbesondere da, wie oben erwähnt, die Augenfolgebewegungen nicht willkürlich erzeugt werden können, sondern der Bewegung des auf dem Bildschirm dargestellten Zeichens, das als Stimulus für das Auge des Nutzers dient, folgen.

Hierzu kann vorzugsweise die Auswerteeinheit den gewünschten Zeitpunkt, zu dem sich aus der Augenbewegungsmetrik des Nutzers die Erkennungsschwelle des Nutzers für das auf dem Bildschirm dargestellte Zeichen, insbesondere die Ortsfrequenz des Musters, während Schritt c) feststellen. Hierzu können bevorzugt Daten zur Erfassung der Augenbewegung des Nutzers, welche die Videokamera aufgenommen und an die Auswerteeinheit weitergeleitet hat, dazu verwendet werden, um die Blickrichtung des Nutzers auf das auf dem Bildschirm dargestellte Zeichen zu ermitteln. Im Falle einer zunehmend Abnahme eines der Parameter des Zeichens, insbesondere der Ortsfrequenz in dem periodischen Muster, wird die Augenfolgebewegung des Nutzers solange der Bewegung des Zeichens über den Bildschirm entsprechen, solange der Nutzer das auf dem Bildschirm dargestellte Zeichen erkennen kann. Ist ein Zeitpunkt erreicht, zu dem der Nutzer das auf dem Bildschirm dargestellte Zeichen, insbesondere das periodische Muster, gerade nicht mehr erkennen kann und das somit nicht mehr als Stimulus für das Auge des Nutzers wirken kann, so weicht die Augenfolgebewegung des Nutzers von der Bewegung des Zeichens auf dem Bildschirm ab. Wird umgekehrt der Zeitpunkt erreicht, zu dem der Nutzer das auf dem Bildschirm dargestellte Zeichen, insbesondere das periodische Muster, erstmals gerade erkennen kann und das somit erstmals als Stimulus für das Auge des Nutzers wirken kann, so wird die Augenfolgebewegung des Nutzers nun beginnen, der Bewegung des Zeichens auf dem Bildschirm zu folgen. Unabhängig von der Art der Ausgestaltung kann hierbei bevorzugt eine Schwelle festgesetzt werden, mittels der ein Grad der Abweichung der Augenfolgebewegung des Nutzers von der Bewegung des Zeichens auf dem Bildschirm als der Zeitpunkt gemäß Schritt c) festgelegt wird. Der Zeitpunkt, zu dem die Abweichung die festgelegte Schwelle überschreitet oder unterschreitet, stellt hierbei den gemäß Schritt c) gesuchten Zeitpunkt dar.

Gemäß Schritt d) erfolgt, vorzugsweise im Anschluss an die Feststellung des Zeitpunkts, bevorzugt in der Auswerteeinheit, die Bestimmung eines Wertes für den Refraktionsfehler des Auges des Nutzers aus dem Wert des Parameters, der zu dem festgestellten Zeitpunkt dazu verwendet wurde, den ausgewählten Parameter des Zeichens auf dem Bildschirm einzustellen. In der oben beschriebenen bevorzugten Ausgestaltung kann der Wert für den Refraktionsfehler aus der zu dem Zeitpunkt festgelegten Ortsfrequenz des Musters, welche auch eine Grenzfrequenz des Musters sein kann, bestimmt werden, zu der sich aus der Beobachtung der Augenbewegungsmetrik des Nutzers während Schritt c) die Erkennungsschwelle des Nutzers für das auf dem Bildschirm dargestellte Zeichen ergibt. Die "Grenzfrequenz" bezeichnet diejenige Ortsfrequenz des Musters, bei welcher die Kontrastempfindlichkeit Null wird oder der Kontrast des Stimulus maximal. Diese Frequenz kann auch als Auflösungsgrenze des visuellen Systems betrachtet werden.

In einer besonders bevorzugten Ausgestaltung der vorliegenden Erfindung kann aus dem zu dem Zeitpunkt festgelegten Parameter zunächst eine Kontrastempfindlichkeit des Auges des Nutzers ermittelt werden. Der Begriff "Kontrastempfindlichkeit" des Auges definiert hierbei ein Maß zur Unterscheidung verschiedener Grauabstufungen als Kehrwert des kleinsten, gerade noch wahrnehmbaren Unterschiedes zweier Grauwerte. Die Begriffe "Sehschärfe" und "Auflösungsvermögen" des Auges des Nutzers geben hierbei jeweils ein Maß für einen räumlichen Abstand zwischen zwei Punkten, den das Auge des Nutzers noch als unterscheidbar wahrnehmen kann. In der oben beschriebenen bevorzugten Ausgestaltung kann die Kontrastempfindlichkeit mittels eines periodischen Musters in Form von periodisch nebeneinander angeordneten Streifen, das auch als "sinusförmiges Gitter" oder als "Gabor Patch" bezeichnet wird, ermittelt werden. In einer bevorzugten Darstellung kann hierzu eine Kontrastempfindlichkeitsfunktion festgelegt werden, in der die Kontrastempfindlichkeit gegenüber der Ortsfrequenz des periodischen Musters aufgetragen ist. Diese Funktion nimmt jeweils einen unterschiedlichen Verlauf für eine ausgewählte Defokussierung des Auges des Nutzers. Aus der Feststellung, welchen Verlauf die Kontrastempfindlichkeit des Auges des Nutzers aufweist, kann auf die vorliegende Defokussierung des Auges und die sphärozylindrische Ausgestaltung des Brillenglases, die zu einem weitgehenden Ausgleich der Refraktionsfehler des Auges und damit zu einer möglichst optimalen Bildqualität für den Nutzer führt, bestimmt werden und hieraus der Wert für den Refraktionsfehler des Auges des Nutzers ermittelt werden. Für weitere Einzelheiten in Bezug auf die Kontrastempfindlichkeitsfunktion und die entsprechende Bestimmung des Wertes für den Refraktionsfehler wird auf A. Leube et al., Individual neural transfer function affects the prediction of subjective depth of focus, Scientific Reports 2018, 8(1), 1919, und auf die Ausführungsbeispiele verwiesen.

Insbesondere kann aus einer Bestimmung der bei dem Nutzer auftretenden Refraktionsfehler somit eine sphärozylindrische Linse ermittelt werden, die als Brillenglas dazu benutzt wird, die als Defokussierung des Auges auftretenden Refraktionsfehlern derart zu kompensieren, dass eine möglichst optimale Bildqualität für den Nutzer erzielt werden kann. Zur Beschreibung der sphärozylindrischen Linse eignen sich verschiedene Darstellungsweisen. Die Norm legt in Abschnitt 11.2 einen so genannten "sphärischen Brechwert" fest, welcher als Wert für einen Scheitelbrechwert eines Brillenglases mit sphärischer Wirkung oder für den jeweiligen Scheitelbrechwert in einem von zwei Hauptschnitten des Brillenglases mit astigmatischer Wirkung definiert ist. Nach der Norm, 9.7.1 und 9.7.2 ist der "Scheitelbrechwert" als Kehrwert einer paraxialen Schnittweite eines bildseitigen Brennpunktes, jeweils gemessen in Metern, festgelegt. Das sphärozylindrische Brillenglas mit astigmatischer Wirkung vereinigt gemäß der Norm, Abschnitt 12, ein paraxiales, paralleles Lichtbündel in zwei getrennten, zueinander senkrecht stehenden Brennlinien, und besitzt daher nur in den beiden Hauptschnitten einen sphärischen Scheitelbrechwert. Die "astigmatische Wirkung" ist hierbei durch Zylinderstärke und Achslage festgelegt. Hierbei stellt die "Zylinderstärke" gemäß der Norm, 12.5 den Betrag einer "astigmatischen Differenz" dar, welche die Differenz zwischen den Scheitelbrechwerten in den beiden Hauptschnitten angibt. Die "Achslage" bezeichnet gemäß der Norm, 12.6 eine Richtung des Hauptschnittes, dessen Scheitelbrechwert als Referenzwert herangezogen wird. Schließlich wird nach der Norm, 12.8 die "Stärke" des Brillenglases mit astigmatischer Wirkung mittels drei Werten, umfassend die Scheitelbrechwerte jedes der beiden Hauptschnitte und die Zylinderstärke, angegeben.

Nach L. N. Thibos, W. Wheeler und D. Horner (1997), Power Vectors: An Application of Fourier Analysis to the Description and Statistical Analysis of Refractive Error, Optometry and Vision Science 74 (6), S. 367-375, eignet sich zur Beschreibung einer beliebigen sphärozylindrischen Linse die Angabe eines "Brechkraftvektors" (*power vector*), welcher durch genau einen Punkt in einem dreidimensionalen dioptrischen Raum beschrieben werden kann, wobei der dreidimensionale dioptrische Raum durch Koordinaten aufgespannt werden kann, welche der mittleren sphärischen Brechkraft und der Zylinderstärke sowie der dazugehörigen Achslage entsprechen bzw. damit korreliert sind. In einer besonderen Ausgestaltung kann das Zeichen, insbesondere das periodische Muster, zunächst in einer ersten Richtung und anschließend in einer zweiten Richtung, die senkrecht zu der ersten Richtung angeordnet sein kann, dargestellt werden. Auf diese Weise können für das sphärozylindrische Brillenglas mit astigmatischer Wirkung nacheinander die Scheitelbrechwerte jedes der beiden Hauptschnitte, die senkrecht zueinander stehen, ermittelt werden.

Somit kann in besonders vorteilhafter Weise auf einen psychophysischen Algorithmus zur Bestimmung des Refraktionsfehlers des Auges verzichtet werden. Hierbei bezeichnet der Begriff des "psychophysischen Algorithmus'" eine Vorgehensweise, welche auf gesetzmäßigen Wechselbeziehungen zwischen subjektivem psychischem, mentalem Erleben des Nutzers und quantitativ messbaren, objektiven physikalischen Stimuli als den Auslöser für das Erleben des Nutzers basiert. Vielmehr wird erfindungsgemäß eine Messgröße in Form einer Erfassung der Augenbewegungsmetrik verwendet, aus der sich Schlüsse auf die Reaktion des Nutzers in Abhängigkeit von einem sich festen oder bewegenden Zeichen, zum Beispiel einer sich zeitlich verändernden Ortsfrequenz ziehen lassen.

In einem weiteren Aspekt betrifft die vorliegende Erfindung ein Computerprogramm zur Bestimmung eines Refraktionsfehlers eines Auges eines Nutzers, wobei das Computerprogramm dazu eingerichtet ist, die Bestimmung des Refraktionsfehlers des Auges des Nutzers gemäß dem hierin beschriebenen Verfahren zur Bestimmung eines Refraktionsfehlers eines Auges eines Nutzers durchzuführen.

In einem weiteren Aspekt betrifft die vorliegende Erfindung ein Verfahren zur Herstellung eines Brillenglases, wobei die Herstellung des Brillenglases durch Bearbeitung eines Brillenglasrohlings erfolgt, wobei der Brillenglasrohling anhand von Zentrierdaten bearbeitet wird, wobei die Zentrierdaten Anweisungen zum Ausgleich des Refraktionsfehlers des Auges des Nutzers umfassen, wobei eine Bestimmung des Refraktionsfehlers des Auges des Nutzers gemäß dem hierin beschriebenen Verfahren zur Bestimmung eines Refraktionsfehlers eines Auges eines Nutzers erfolgt.

In einem weiteren Aspekt betrifft die vorliegende Erfindung eine Vorrichtung zur Bestimmung des Refraktionsfehlers des Auges des Nutzers. Erfindungsgemäß umfasst die Vorrichtung
- einen Bildschirm, der zur Darstellung eines Zeichen und einer Veränderung eines Parameters des Zeichens eingerichtet ist;
- eine Videokamera, die zur Erfassung einer Augenbewegungsmetrik des Auges des Nutzers in Abhängigkeit von dem auf dem Bildschirm dargestellten Zeichen eingerichtet ist; und
- eine Auswerteeinheit, die zur Feststellung eines Zeitpunkts, zu dem sich aus der Augenbewegungsmetrik des Auges des Nutzers eine Erkennungsschwelle des Nutzers für das auf dem Bildschirm dargestellte Zeichen ergibt, und zur Bestimmung eines Wertes für den Refraktionsfehler des Auges des Nutzers aus dem zu dem Zeitpunkt festgelegten Parameter eingerichtet ist.

In einer besonders bevorzugten Ausgestaltung kann die Auswerteeinheit weiterhin über eine Einrichtung zur Erfassung eines Abstands des Auges des Nutzers von dem Bildschirm oder der Videokamera verfügen. Hierzu kann durch Bildverarbeitung einer Bildfolge, welche von der Videokamera insbesondere von der Augenpartie des Nutzers aufgenommen wurde, aus einer Bestimmung einer Pupillendistanz zwischen der Videokamera und dem Auge des Nutzers insbesondere dann, wenn eine Kalibrierung der Pixel der Kamera in räumlichen Einheiten vorliegt, eine Ermittlung des Pupillendurchmessers des Auges des Nutzers ausgeführt werden. In einer bevorzugten Ausgestaltung können mindestens zwei Kameras vorgesehen sein, die gemeinsam in Form einer Stereokamera angeordnet sind und daher zur Erfassung des Abstands des Auges des Nutzers von dem Bildschirm eingerichtet sind. Alternativ oder zusätzlich kann die Vorrichtung über einen Entfernungsmesser verfügen, der zur Bestimmung der Pupillendistanz zwischen der Videokamera und dem Auge des Nutzers eingerichtet ist.

Für Definitionen und optionale Ausgestaltungen des Computerprogramms und der Vorrichtung zur Bestimmung eines Refraktionsfehlers eines Auges eines Nutzers sowie des Verfahrens zur Herstellung eines Brillenglases einschließlich der darin aufgeführten Merkmale wird auf die oben oder unten stehende Beschreibung des Verfahrens zur Bestimmung eines Refraktionsfehlers eines Auges eines Nutzers verwiesen.

Die erfindungsgemäße Vorrichtung und die vorliegenden Verfahren können insbesondere bei einer Auswahl einer neuen Brille für den Nutzer eingesetzt werden. Alternativ oder zusätzlich ist jedoch auch ein Einsatz bei einer Überprüfung einer bereits vorhandenen Brille des Nutzers möglich. Im letzteren Falle kann der Nutzer die Brille bei der Durchführung des Verfahrens zur Bestimmung des Refraktionsfehlers tragen und die Abweichungen gegenüber einer optimalen Refraktion durch das optische System, umfassend das Auge und die Brille des Nutzers, feststellen. Der hierbei bestimmte Wert kann zudem für die Auswahl und Herstellung einer weiteren Brille, die z.B. als Zweitbrille oder als Ersatzbrille dienen kann, eingesetzt werden.

Die erfindungsgemäße Vorrichtung und die vorliegenden Verfahren weisen gegenüber herkömmlichen Vorrichtungen und Verfahren zahlreiche Vorteile auf. Damit kann eine objektive Ermittlung der Korrektion eines Refraktionsfehlers eines Auges eines Nutzers ohne Spezialgeräte erfolgen, insbesondere ohne dass ein subjektives Feedback durch den Nutzer, z.B. in Form einer manuellen oder akustischen Eingabe in die Vorrichtung, erforderlich wäre. Die vorgeschlagene Vorrichtung ist nicht an einen Ort gebunden und kann hierbei insbesondere auch von Laien benutzt werden. Weiterhin wird hierbei in vorteilhafter Weise, die mittels der Erfassung der Augenbewegungsmetrik bestimmte Kontrastempfindlichkeit für die Ermittlung der Korrektion verwendet, um so auf einfache Weise den Refraktionsfehler des Auges des Nutzers zu bestimmen.

Zusammenfassend sind im Rahmen der vorliegenden Erfindung folgende Ausführungsformen besonders bevorzugt:
Ausführungsform 1. Verfahren zur Bestimmung eines Refraktionsfehlers eines Auges eines Nutzers, wobei das Verfahren die folgenden Schritte umfasst:
   a) Darstellen eines Zeichens auf einem Bildschirm, wobei ein Parameter des auf dem Bildschirm dargestellten Zeichens verändert wird;
   b) Erfassen einer Augenbewegungsmetrik des Auges des Nutzers in Abhängigkeit von dem auf dem Bildschirm dargestellten Zeichen;
   c) Feststellen eines Zeitpunkts, zu dem sich aus der Augenbewegungsmetrik des Auges des Nutzers eine Erkennungsschwelle des Nutzers für das auf dem Bildschirm dargestellte Zeichen für den Nutzer ergibt; und
   d) Bestimmen eines Wertes für den Refraktionsfehler des Auges des Nutzers aus dem zu dem Zeitpunkt festgelegter Parameter.
Ausführungsform 2. Verfahren nach der vorangehenden Ausführungsform, wobei der Refraktionsfehlers für ein Auge oder für beide Augen des Nutzers bestimmt wird.
Ausführungsform 3. Verfahren nach einer der vorangehenden Ausführungsformen, wobei der Parameter des auf dem Bildschirm dargestellten Zeichens verändert wird, während das auf dem Bildschirm dargestellte Zeichen eine Bewegung über den Bildschirm ausführt.
Ausführungsform 4. Verfahren nach der vorangehenden Ausführungsform, wobei die Bewegung des auf dem Bildschirm dargestellten Zeichens kontinuierlich oder in Sprüngen erfolgt.
Ausführungsform 5. Verfahren nach einer der beiden vorangehenden Ausführungsformen, wobei der Zeitpunkt dadurch festgestellt wird, dass die Augenbewegungsmetrik des Nutzers der Bewegung des auf dem Bildschirm dargestellten Zeichens gerade noch folgt oder gerade erst folgt.
Ausführungsform 6. Verfahren nach einer der vorangehenden Ausführungsformen, wobei Schritt b) für verschiedene Werte des Parameters wiederholt wird.
Ausführungsform 7. Verfahren nach der vorangehenden Ausführungsform, wobei Schritt b) solange für verschiedene Werte des Parameters wiederholt wird, bis der Zeitpunkt gemäß Schritt c) festgestellt wird.
Ausführungsform 8. Verfahren nach einer der vorangehenden Ausführungsformen, wobei sich die Augenbewegungsmetrik bezieht auf: eine Augenfolgebewegung; eine Augenbewegung, die sich auf Mikrosakkaden bezieht; oder einen optokinetischen Nystagmus.
Ausführungsform 9. Verfahren nach der vorangehenden Ausführungsform, wobei die Augenbewegung, die sich auf Mikrosakkaden bezieht, ausgewählt ist aus: einer Mikrosakkadenrichtung oder einer Mikrosakkadenrate.
Ausführungsform 10. Verfahren nach der vorangehenden Ausführungsform, wobei die Augenbewegungsmetrik, die sich auf Mikrosakkaden bezieht, als Maß dafür verwendet wird, um festzustellen, ob der Nutzer das Zeichen als Stimulus erkannt hat oder nicht.
Ausführungsform 11. Verfahren nach der vorangehenden Ausführungsform, wobei die Augenbewegungsmetrik, die sich auf Sakkaden bezieht, ausgewählt ist aus einer Genauigkeit der ausgeführten Sakkade in Bezug auf die räumliche Position des Stimulus auf dem Bildschirm, welche als Maß dafür verwendet wird, um festzustellen, ob ein Nutzer das Zeichen als Stimulus erkannt hat oder nicht.
Ausführungsform 12. Verfahren nach der vorangehenden Ausführungsform, wobei die Augenbewegungsmetrik, die sich auf den optokinetischen Nystagmus bezieht, ausgewählt ist aus: einer Phasenübereinstimmung zwischen der Augenfolgenbewegung und der Bewegung des Stimulus auf dem Bildschirm, die als Maß dafür verwendet wird, um festzustellen, ob ein Nutzer das Zeichen als Stimulus erkannt hat oder nicht. Ausführungsform 13. Verfahren nach einer der vorangehenden Ausführungsformen, wobei aus dem zu dem Zeitpunkt festgelegten Parameter zunächst ein Wert für eine Kontrastempfindlichkeit des Auges des Nutzers ermittelt wird.
Ausführungsform 14. Verfahren nach der vorangehenden Ausführungsform, wobei eine Kontrastempfindlichkeitsfunktion festgestellt wird, welche an die ermittelten Werte der Kontrastempfindlichkeit des Auges des Nutzers angepasst ist.
Ausführungsform 15. Verfahren nach der vorangehenden Ausführungsform, wobei aus der festgestellten Kontrastempfindlichkeitsfunktion eine Defokussierung des Auges des Nutzers ermittelt wird,
Ausführungsform 16. Verfahren nach der vorangehenden Ausführungsform, wobei aus der Defokussierung des Auges des Nutzers der Wert für den Refraktionsfehler des Auges des Nutzers bestimmt wird.
Ausführungsform 17. Verfahren nach einer der vier vorangehenden Ausführungsformen, wobei der Wert für den Refraktionsfehler des Auges des Nutzers aus der Kontrastempfindlichkeit des Auges des Nutzers bestimmt wird.
Ausführungsform 18. Verfahren nach einer der vorangehenden Ausführungsformen, wobei das Zeichen ein periodisches Muster ist oder umfasst.
Ausführungsform 19. Verfahren nach der vorangehenden Ausführungsform, wobei der Parameter des auf dem Bildschirm dargestellten Musters mindestens eine Ortsfrequenz ist oder umfasst.
Ausführungsform 20. Verfahren nach einer der vorangehenden Ausführungsformen, wobei das Zeichen zunächst in einer ersten Richtung in Bezug auf eine Orientierung des Bildschirms dargestellt wird und anschließend in einer gegen die erste Richtung veränderten zweiten Richtung dargestellt wird.
Ausführungsform 21. Verfahren nach der vorangehenden Ausführungsform, wobei nacheinander die Scheitelbrechwerte jedes der beiden Hauptschnitte, die senkrecht zueinander stehen, für ein sphärozylindrisches Brillenglas mit astigmatischer Wirkung ermittelt werden.
Ausführungsform 22. Verfahren nach einer der vorangehenden Ausführungsformen, wobei zusätzlich ein Abstand des Auges des Nutzers von dem Bildschirm erfasst wird.
Ausführungsform 23. Verfahren nach der vorangehenden Ausführungsform, wobei Abstand des Auges des Nutzers von dem Bildschirm eine Pupillendistanz ist.
Ausführungsform 24. Verfahren nach einer der beiden vorangehenden Ausführungsformen, wobei der Abstand mittels eines Entfernungsmessers erfasst wird.
Ausführungsform 25. Verfahren nach einer der drei vorangehenden Ausführungsformen, wobei der Abstand mittels der Videokamera erfasst wird.
Ausführungsform 26. Verfahren nach einer der vier vorangehenden Ausführungsformen, wobei der Abstand mittels mindestens zwei Kameras, die gemeinsam zur Erfassung des Abstands eingerichtet sind, erfasst wird.
Ausführungsform 27. Verfahren nach der vorangehenden Ausführungsform, wobei die mindestens zwei Kameras gemeinsam in Form einer Stereokamera angeordnet sind.
Ausführungsform 28. Verfahren nach einer der vorangehenden Ausführungsformen, wobei das Verfahren ausgeführt wird, während der Nutzer eine Brille trägt.
Ausführungsform 29. Computerprogramm zur Bestimmung eines Refraktionsfehlers eines Auges eines Nutzers, wobei das Computerprogramm dazu eingerichtet ist, die Schritte nach einer der vorangehenden Ausführungsformen durchzuführen.
Ausführungsform 30. Verfahren zur Herstellung mindestens eines Brillenglases, wobei die Herstellung des Brillenglases durch Bearbeitung eines Brillenglasrohlings erfolgt, wobei der Brillenglasrohling anhand von Zentrierdaten bearbeitet wird, wobei die Zentrierdaten Anweisungen zum Ausgleich des Refraktionsfehlers des Auges des Nutzers umfassen, wobei eine Bestimmung des Refraktionsfehlers des Auges des Nutzers gemäß den Schritten nach einer der vorangehenden Ausführungsformen in Bezug auf das Verfahren zur Bestimmung eines Refraktionsfehlers eines Auges eines Nutzers erfolgt.
Ausführungsform 31. Vorrichtung zur Bestimmung des Refraktionsfehlers des Auges des Nutzers, wobei die Vorrichtung umfasst:
   - einen Bildschirm, der zur Darstellung eines Zeichen und einer Veränderung eines Parameters des Zeichens eingerichtet ist;
   - eine Videokamera, die zur Erfassung einer Augenbewegungsmetrik des Auges des Nutzers in Abhängigkeit von dem auf dem Bildschirm dargestellten Zeichen eingerichtet ist; und
   - eine Auswerteeinheit, die zur Feststellung eines Zeitpunkts, zu dem sich aus der Augenbewegungsmetrik des Auges des Nutzers eine Erkennungsschwelle des Nutzers für das auf dem Bildschirm dargestellte Zeichen ergibt, und zur Bestimmung eines Wertes für den Refraktionsfehler des Auges des Nutzers aus dem zu dem Zeitpunkt festgelegten Parameter eingerichtet ist.
Ausführungsform 32. Vorrichtung nach der vorangehenden Ausführungsform, wobei die Vorrichtung weiterhin einen Entfernungsmesser umfasst, wobei der Entfernungsmesser weiterhin dazu eingerichtet ist, einen Abstand zwischen der Vorrichtung und dem Auge des Nutzers zu bestimmen.
Ausführungsform 33. Vorrichtung nach einer der beiden vorangehenden Ausführungsform, wobei die Videokamera dazu eingerichtet ist, eine Aufnahme eines Bildes des Auges des Nutzers vorzunehmen, wobei die Auswerteeinheit weiterhin dazu eingerichtet ist, durch Bildverarbeitung des Bildes des Auges des Nutzers den Abstand zwischen der Vorrichtung und dem Auge des Nutzers zu bestimmen.
Ausführungsform 34. Vorrichtung nach einer drei vorangehenden Ausführungsformen, wobei mindestens zwei Kameras vorgesehen sind, die gemeinsam zur Erfassung des den Abstand zwischen der Vorrichtung und dem Auge des Nutzers eingerichtet sind.
Ausführungsform 35. Vorrichtung nach der vorangehenden Ausführungsform, wobei die mindestens zwei Kameras gemeinsam in Form einer Stereokamera angeordnet sind.
Ausführungsform 36. Vorrichtung nach einer der fünf vorangehenden Ausführungsformen, wobei die Vorrichtung als mobiles Kommunikationsgerät ausgestaltet ist, wobei das mobile Kommunikationsgerät den Bildschirm, die Videokamera, die Auswerteeinheit und optional den Entfernungsmesser umfasst.
Ausführungsform 37. Vorrichtung nach der vorangehenden Ausführungsform, wobei das mobile Kommunikationsgerät als Smartphone ausgestaltet ist.

In einem weiteren Aspekt können das vorstehend beschriebene Verfahren und/oder die vorstehend beschriebene Vorrichtung und/oder das vorstehend beschriebene Computerprogramm zusammen mit wenigstens einem weiteren Verfahren und/oder wenigstens einer weiteren Vorrichtung und/oder einem weiteren Computerprogramm zur Anwendung kommen. Bei diesem wenigstens einen weiteren Verfahren kann es sich um ein beispielsweise um ein Verfahren zur Bestimmung eines Refraktionsfehlers eines Auges eines Nutzers handeln, wobei dieses Verfahren die folgenden Schritte umfasst:
a) Darstellen eines Zeichens auf einem Bildschirm, wobei ein Parameter des auf dem Bildschirm dargestellten Zeichens verändert wird;
b) Erfassen einer Reaktion des Nutzers in Abhängigkeit von dem auf dem Bildschirm dargestellten Zeichen;
c) Feststellen eines Zeitpunkts, zu dem sich aus der Reaktion des Nutzers eine Erkennbarkeit des auf dem Bildschirm dargestellten Zeichens für den Nutzer ergibt; und
d) Bestimmen eines Wertes für den Refraktionsfehler des Auges des Nutzers aus dem zu dem Zeitpunkt festgelegten Parameter, wobei das auf dem Bildschirm dargestellte Zeichen ein periodisches Muster ist, wobei der Parameter des auf dem Bildschirm dargestellten Musters mindestens eine Ortsfrequenz umfasst, und der Wert für den Refraktionsfehler aus der zu dem Zeitpunkt festgelegten Ortsfrequenz des Musters bestimmt wird.

Alternativ oder zusätzlich zu dem vorstehend beschriebenen Verfahren kann es sich bei dem wenigstens einen weiteren Verfahren beispielsweise auch um ein Verfahren zur Bestimmung mindestens eines optischen Parameters eines Brillenglases handeln, wobei dieses Verfahren folgende Schritte umfasst:
a) Aufnehmen eines Bildes unter Verwendung eines Brillenglases; und
b) Ermitteln mindestens eines optischen Parameters des Brillenglases mittels Bildverarbeitung des Bildes, wobei das Bild eine die Augen einschließende Augenpartie und/oder eine an die Augen angrenzende Gesichtspartie eines Nutzers des Brillenglases umfasst.

Alternativ oder zusätzlich zu den vorstehend beschriebenen Verfahren kann es sich bei dem wenigstens einen weiteren Verfahren beispielsweise auch um ein Verfahren zum Vermessen der Brechkraftverteilung eines linken und/oder eines rechten Brillenglases in einer Brillenfassung handeln, in welchem in einem ersten Schritt mittels wenigstens einer Bilderfassungseinrichtung wenigstens eine erste Abbildung einer Szene aus wenigstens einer ersten Aufnahmeposition erfasst wird, wobei diese wenigstens eine erste Abbildung wenigstens zwei Strukturpunkte aufweist und ein linkes und/oder ein rechtes Brillenglas in einer Brillenfassung mit einem ein Koordinatensystem der Brillenfassung definierenden Abschnitt der Brillenfassung enthält, wobei der wenigstens eine Abbildungsstrahlengang für jeden dieser wenigstens zwei Strukturpunkte das erste und/oder das zweite Brillenglas der Brillenfassung jeweils mindestens einmal passiert und mindestens einmal nicht passiert. Jeder Abbildungsstrahlengang umfasst die Position des Strukturpunkts sowie den in die wenigstens eine Bilderfassungseinrichtung einfallenden Hauptstrahl. In einem weiteren Schritt, der zeitlich vor oder nach dem ersten Schritt liegen kann, wird wenigstens eine weitere Abbildung der Szene ohne das erste und/oder das zweite Brillenglas der Brillenfassung oder ohne die Brillenfassung enthaltend das erste und/oder das zweite Brillenglas mit denselben wenigstens zwei Strukturpunkten der ersten Abbildung einer Szene mittels wenigstens einer Bilderfassungseinrichtung aus der ersten Aufnahmeposition oder aus wenigstens einer von der ersten Aufnahmeposition verschiedenen weiteren Aufnahmeposition erfasst. Die wenigstens eine Bilderfassungseinrichtung kann in dem weiteren Schritt identisch oder verschieden zu der wenigstens einen Bilderfassungseinrichtung aus dem ersten Schritt sein. Bevorzugt ist die wenigstens eine Bilderfassungseinrichtung in dem weiteren Schritt identisch zu der wenigstens einen Bilderfassungseinrichtung aus dem ersten Schritt. Darauf werden in einem Schritt des Berechnens die Koordinaten dieser wenigstens zwei Strukturpunkte in einem zu dem Koordinatensystem der Brillenfassung referenzierten Koordinatensystem der Abbildung dieser Szene aus dem jeweiligen wenigstens einen Strahlengang dieser wenigstens zwei Strukturpunkte, welcher das linke und/oder rechte Brillenglas jeweils nicht passiert hat, und der wenigstens einen weiteren Abbildung der Szene mittels Bildauswertung bestimmt. Im Anschluss daran wird die Brechkraftverteilung in einem Schritt des Bestimmens einer Brechkraftverteilung für wenigstens einen Abschnitt des linken Brillenglases in dem Koordinatensystem der Brillenfassung und/oder in einem Schritt des Bestimmens einer Brechkraftverteilung für wenigstens einen Abschnitt des rechten Brillenglases in dem Koordinatensystem der Brillenfassung, jeweils aus den Abbildungsstrahlungsgängen, welche das jeweilige Brillenglas passiert haben, bestimmt.

Alternativ oder zusätzlich zu den vorstehend beschriebenen Verfahren kann es sich bei dem wenigstens einen weiteren Verfahren beispielsweise auch um ein Verfahren zum Vermessen der Brechkraftverteilung eines linken und/oder eines rechten Brillenglases in einer Brillenfassung handeln, in welchem in einem ersten Schritt mittels wenigstens einer Bilderfassungseinrichtung wenigstens eine erste Abbildung einer Szene aus wenigstens einer ersten Aufnahmeposition erfasst wird, wobei diese wenigstens eine erste Abbildung wenigstens zwei Strukturpunkte aufweist und ein linkes und/oder ein rechtes Brillenglas in einer Brillenfassung mit einem ein Koordinatensystem der Brillenfassung definierenden Abschnitt der Brillenfassung enthält, wobei der wenigstens eine Abbildungsstrahlengang für jeden dieser wenigstens zwei Strukturpunkte das erste und/oder das zweite Brillenglas der Brillenfassung jeweils mindestens einmal passiert und mindestens einmal nicht passiert. Jeder Abbildungsstrahlengang umfasst die Position des Strukturpunkts sowie den in die wenigstens eine Bilderfassungseinrichtung einfallenden Hauptstrahl. In einem weiteren Schritt, der zeitlich vor oder nach dem ersten Schritt liegen oder gleichzeitig mit dem ersten Schritt erfolgen kann, wird wenigstens eine weitere Abbildung der Szene mit dem linken und/oder dem rechten Brillenglas in einer Brillenfassung und mit einem ein Koordinatensystem der Brillenfassung definierenden Abschnitt der Brillenfassung mittels wenigstens einer Bilderfassungseinrichtung aus wenigstens einer von der ersten Aufnahmeposition verschiedenen weiteren Aufnahmeposition mit wenigstens einem Abbildungsstrahlengang für dieselben wenigstens zwei in der ersten Abbildung erfassten Strukturpunkte erfasst, wobei dieser wenigstens eine Abbildungsstrahlengang das erste und/oder das zweite Brillenglas der Brillenfassung jeweils mindestens einmal passiert und mindestens einmal nicht passiert. Darauf werden in einem weiteren Schritt die Koordinaten der wenigstens zwei Strukturpunkte in einem zu dem Koordinatensystem der Brillenfassung referenzierten Koordinatensystem der Szene aus dem jeweiligen wenigstens einen Strahlengang dieser wenigstens zwei Strukturpunkte, welcher das linke und/oder rechte Brillenglas jeweils nicht passiert hat und der wenigstens einen weiteren Abbildung der Szene mittels Bildauswertung berechnet. Anschließend wird die Brechkraftverteilung für wenigstens einen Abschnitt des linken Brillenglases in dem Koordinatensystem der Brillenfassung berechnet und/oder es wird die Brechkraftverteilung für wenigstens einen Abschnitt des rechten Brillenglases in dem Koordinatensystem der Brillenfassung, jeweils aus den Abbildungsstrahlengängen, welche das jeweilige Brillenglas passiert haben, bestimmt.

Bevorzugt wird in den beiden vorstehenden Verfahren zum Vermessen der Brechkraftverteilung eines linken und/oder eines rechten Brillenglases, vorzugsweise in einer Brillenfassung, eine Vielzahl von Strukturpunkten in der jeweils ersten Abbildung einer Szene aus jeweils wenigstens einer ersten Aufnahmeposition erfasst und die jeweils darauf folgenden Schritte anhand dieser jeweiligen Vielzahl von Strukturpunkten vorgenommen. Unter einer Vielzahl von Strukturpunkten werden bevorzugt wenigstens 10, weiter bevorzugt wenigstens 100, besonders bevorzugt wenigstens 1000 und ganz besonders bevorzugt wenigstens 10000 Strukturpunkte verstanden. Insbesondere ist eine Vielzahl von Strukturpunkten ≥100 Strukturpunkte und ≤1000 Strukturpunkte.

Alternativ oder zusätzlich zu den vorstehend beschriebenen Verfahren kann es sich bei dem wenigstens einen weiteren Verfahren beispielsweise auch um ein Verfahren zur Bestimmung der Brechkraftverteilung eines Brillenglases handeln, welches aus dem Größen- und/oder Formvergleich der Abbildung des vorderen Augenabschnittes für eine bestimmte Durchblickrichtung eine lokale Brechkraft ermöglicht. Hierzu wird wenigstens eine Aufnahme des vorderen Augenabschnittes mit und ohne davor befindlichem Brillenglas durchgeführt und die Aufnahme mit und ohne Brillenglas jeweils miteinander verglichen.

In einer übergeordneten Anwendung können die verschiedenen vorstehend beschriebenen Verfahren, d.h. das erfindungsgemäße Verfahren sowie das wenigstens eine weitere Verfahren, kombiniert werden, um beispielsweise aus einem Vergleich der jeweils erhaltenen Ergebnisse eine höhere Genauigkeit oder eine Plausibiltätsüberprüfung der in den einzelnen Verfahren erhaltenen Ergebnisse zu erhalten. Die verschiedenen vorstehend beschriebenen Verfahren können nacheinander oder gleichzeitig in der übergeordneten Anwendung erfolgen. Erfolgen die verschiedenen Verfahren nacheinander kann deren Reihenfolge unabhängig voneinander sein und/oder es kann sich um eine beliebige Reihenfolge handeln. Erfolgen die verschiedenen Verfahren nacheinander kann es bevorzugt sein, wenigstens eines der vorstehend beschriebenen Verfahren zur Bestimmung der Brechkraftverteilung zuletzt durchzuführen. Eine übergeordnete Anwendung kann beispielsweise ein die verschiedenen Verfahren umfassendes Computerprogramm sein.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigen:
- Figur 1: ein bevorzugtes Ausführungsbeispiel einer Vorrichtung zur Bestimmung eines Refraktionsfehlers eines Auges eines Nutzers;
- Figur 2: eine schematische Darstellung, in der eine Kontrastempfindlichkeit *ε* des Auges des Nutzers gegenüber einer Ortsfrequenz *f* eines periodischen Musters aufgetragen ist; und
- Figur 3: ein Ablaufdiagramm eines bevorzugten Ausführungsbeispiels eines erfindungsgemäßen Verfahrens zur Herstellung mindestens eines Brillenglases.

### Ausführungsbeispiele

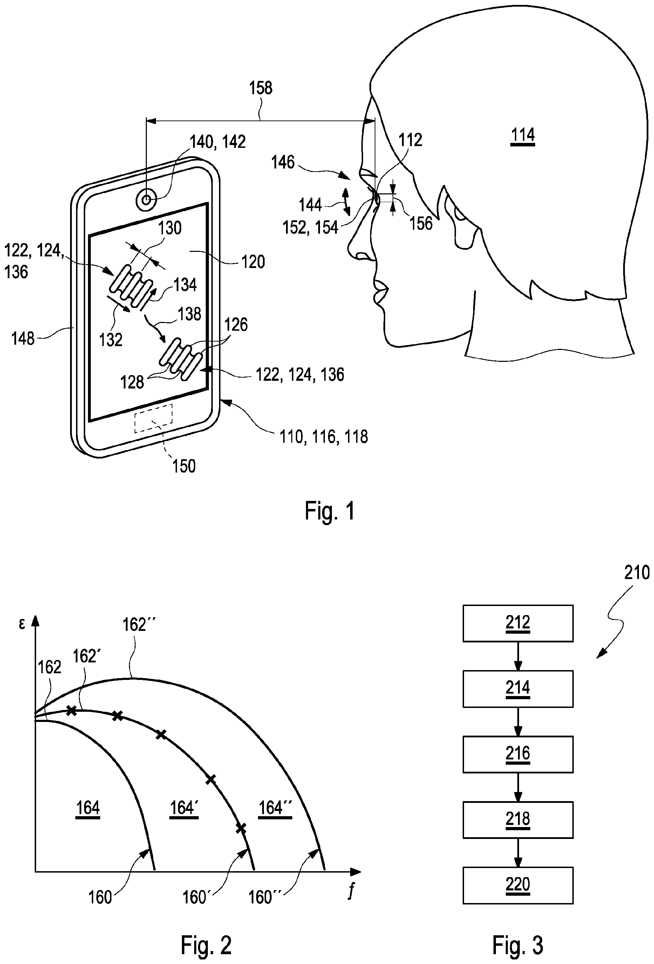

Figur 1 zeigt schematisch ein bevorzugtes Ausführungsbeispiel einer Vorrichtung 110 zur Bestimmung eines Refraktionsfehlers eines Auges 112 eines Nutzers 114. Die Vorrichtung 110 kann hierbei zur Bestimmung des Refraktionsfehlers eines Auges 112 oder beider Augen 112 des Nutzers 114 eingesetzt werden. Die vorgeschlagene Vorrichtung 110 ist in der Darstellung gemäß Figur 1 und in der nachfolgenden Beschreibung - ohne Beschränkung der Allgemeinheit - als mobiles Kommunikationsgerät 116 in Form eines Smartphones 118 ausgeführt. Eine Ausführung der Vorrichtung 110 in Form eines anderen mobilen Kommunikationsgeräts 116, insbesondere als Mobiltelefon (Handy) oder Tablet, ist jedoch ebenfalls denkbar.

Die Vorrichtung 110 umfasst einen Bildschirm 120, der in der Darstellung gemäß Figur 1 im Wesentlichen die Form eines Rechtecks annimmt. Andere Formen des Bildschirms 120 sind jedoch möglich. Der Bildschirm 120 ist zur Darstellung eines Zeichens 122 eingerichtet. In einer bevorzugten Ausführung der vorliegenden Erfindung stellt das Zeichen 122 ein Muster 124 dar, welches über eine graphische Struktur verfügt, die - insbesondere im Gegensatz zu einem Rauschen, das ohne erkennbare Struktur bleibt - über mindestens eine räumlich orientierte Periode verfügt, innerhalb der die Struktur des Musters 124 wiederholt dargestellt ist. Das Muster wird daher auch als periodisches Muster bezeichnet.

Der Bildschirm 120 ist weiterhin zur Darstellung einer Veränderung eines Parameters des auf dem Bildschirm dargestellten Zeichens 122 eingerichtet. Aufgrund einer elektronischen Ansteuerung des Bildschirms 120 auf dem Smartphone 118 kann der ausgewählte Parameter des auf dem Bildschirm dargestellten Musters 124 auf einfache Weise und in einem weiten Rahmen verändert werden. Bei dem hier vorliegenden periodischen Muster 124 kann der Parameter bevorzugt auf eine Eigenschaft einer periodischen Funktion verknüpft sein. Hier kann insbesondere eine Wiederholfrequenz verwendet werden, mit welcher die Struktur derart wiederholt dargestellt werden kann, dass sich durch die Wiederholung gleichartige Punkte oder Bereiche über die Struktur des Musters 124 ausbilden können. In der Darstellung gemäß Figur 1 sind periodische Maxima 126 und Minima 128 als bevorzugte Ausgestaltungen gleichartiger Punkte oder Bereiche des Musters 124 erkennbar. Bei der hier verwendeten periodischen Funktion handelt es sich um eine Sinusfunktion. Andere periodische Funktionen, z.B. eine Cosinusfunktion oder einer Überlagerung einer Cosinusfunktion mit einer Sinusfunktion, sind jedoch denkbar. Der Parameter des gemäß Figur 1 auf dem Bildschirm 120 dargestellten Zeichens mindestens eine Ortsfrequenz des periodischen Musters 124, wobei der Begriff der Ortsfrequenz einen Kehrwert eines räumlichen Abstands 130 zwischen benachbart angeordneten gleichartigen Punkten, insbesondere zwischen benachbarten Maxima 126 oder zwischen benachbarten Minima 128, in einer räumlich periodischen Änderung des Musters 124, bezeichnet. Andere Arten der Bestimmung der Ortsfrequenz aus dem Muster 124 sind jedoch denkbar, beispielsweise aus einem Abstand von Punkten gleicher Intensität.

Wie Figur 1 weiterhin zeigt, umfasst das periodische Muster in dieser besonders bevorzugten Ausführung eine zweidimensionale Überlagerung der periodischen Funktion, insbesondere der Sinusfunktion, welche sich in eine erste Richtung 132 auf der Ausdehnung des Bildschirms 120 erstreckt, und einer konstanten Funktion, welche sich in eine zweite Richtung 134 auf der Ausdehnung des Bildschirms 120 erstreckt, die hier senkrecht zu der ersten Richtung 132 angeordnet ist. Andere Winkel zwischen der ersten Richtung 132 und der zweiten Richtung 134 sind jedoch ebenfalls möglich. Auf diese Weise kann das Muster 124 auf dem Bildschirm 120 in Form von periodisch nebeneinander angeordneten Streifen 136 vorliegen, die auch als "sinusförmiges Gitter" oder "Gabor Patch" bezeichnet werden. Andere Arten von Mustern 124 sind jedoch ebenfalls möglich. Der Nutzer 114 betrachtet auf dem Bildschirm somit das sinusförmige Gitter, das die periodisch nebeneinander angeordneten Streifen 136 mit hohem Kontrast und einer Vielzahl an Ortsfrequenzen umfasst, in einer festgelegten Entfernung. Für die Entfernung kann vorzugsweise ein Wert von 25 cm bis 1 m, besonders bevorzugt von 40 cm bis 75 cm, insbesondere von 50 cm, gewählt werden.

In der in Figur 1 dargestellten besonders bevorzugten Ausführung der vorliegenden Erfindung wird die Ortsfrequenz des auf dem Bildschirm 120 dargestellten Musters 124 verändert, insbesondere während das auf dem Bildschirm 120 dargestellte Muster 122 eine Bewegung 138 über den Bildschirm 120 ausführt. Hierbei kann die Bewegung 138 des auf dem Bildschirm 120 dargestellten Musters 124 kontinuierlich sein. Alternativ kann die Bewegung 138 des Musters 124 auch in Sprüngen über die Ausdehnung des Bildschirms 120 erfolgen. Die Veränderung der Ortsfrequenz des auf dem Bildschirm 120 dargestellten Musters 124 kann insbesondere dadurch erfolgen, dass zu Beginn eine hohe Ortsfrequenz dargestellt wird und diese dann schrittweise verringert wird, oder in dem zu Beginn eine geringe Ortsfrequenz dargestellt wird, die dann schrittweise erhöht wird. Erfindungsgemäß wird die Einstellung des Wertes für die Ortsfrequenz unabhängig vom Nutzer 114 vorgenommen.

Die Vorrichtung 110 umfasst weiterhin eine Videokamera 140, die zur Erfassung einer Augenbewegungsmetrik 144 des Nutzers 114 in Abhängigkeit von dem auf dem Bildschirm 120 dargestellten Muster 124 eingerichtet ist. Wie in Figur 1 schematisch dargestellt, kann es sich bei der Videokamera 140 vorzugsweise um eine Frontkamera 142 des Smartphones 118 handeln. Andere Ausführungen sind jedoch möglich. Mittels der Videokamera 140 kann, insbesondere während der Nutzer 114 das sinusförmige Gitter auf dem Bildschirm 120 des Smartphones 118 betrachtet, ein Bild einer Augenpartie 146 des Nutzers 114 aufgenommen werden. Ändert sich einer der Parameter des Zeichens 122, insbesondere die Ortsfrequenz in dem periodischen Muster 124, kann hierbei ein Zeitpunkt festgestellt werden, an dem das auf dem Bildschirm 120 dargestellte Zeichen 122 nicht mehr als Stimulus für das Auge 112 des Nutzers 114 wirken kann. Umgekehrt kann, falls einer der Parameter des Zeichens 122, insbesondere die Ortsfrequenz in dem periodischen Muster 124, sich ändert, hierbei der Zeitpunkt festgestellt werden, an dem das auf dem Bildschirm dargestellte Zeichen 122 erstmals als Stimulus das Auge 112 des Nutzers 114 wirken kann.

In der in Figur 1 dargestellten besonders bevorzugten Ausgestaltung kann der Zeitpunkt, zu dem sich aus der Augenbewegungsmetrik 144 des Nutzers 114 ergibt, dass eine Erkennungsschwelle für das auf dem Bildschirm 120 dargestellte Zeichen 122 vorliegt, worunter zu verstehen ist, dass der Nutzer 114 das auf dem Bildschirm 120 dargestellte Zeichen 122 gerade noch oder gerade erst wahrnehmen kann, dadurch festgestellt werden, dass die Augenbewegungsmetrik 144 des Nutzers 114 der Bewegung 138 des auf dem Bildschirm 120 dargestellten Zeichens 122 noch folgt oder gerade erst beginnt zu folgen. Wie oben bereits erwähnt, stellt die Augenbewegungsmetrik 144 ein Maß dar, welches mit einer Bewegung des Auges 112 des Nutzers 114 verknüpft ist, wobei die Bewegung des Auges 112 des Nutzers 112 von der Bewegung 138 und der Veränderung des auf dem Bildschirm 120 dargestellten Musters 124 als äußerem auf das Auge 112 des Nutzers 114 einwirkender Stimulus hervorgerufen wird. Bei der von der Videokamera 140 aufgenommenen Augenbewegungsmetrik 144 kann es sich insbesondere um einer Änderung einer Augenfolgebewegung, einer Mikrosakkadenrichtung, einer Mikrosakkadenrate, einer Sakkadengenauigkeit oder eines optokinetischen Nystagmus, die in Folge einer Änderung des Stimulus auftritt, handeln.

Wie weiterhin in Figur 1 schematisch dargestellt, kann die Vorrichtung 110 ein Gehäuse 148 aufweisen, welches eine Auswerteeinheit 150 umfassen kann. Alternativ oder zusätzlich kann die Auswerteeinheit 150 jedoch auch außerhalb des Gehäuses 148 angebracht sein, wobei eine drahtgebundene oder eine drahtlose Verbindung (nicht dargestellt) zwischen der Videokamera 140 und der Auswerteeinheit 150 vorgesehen sein kann. Weitere Arten der Ausführung sind jedoch möglich.

Erfindungsgemäß ist die Auswerteeinheit 150 dazu eingerichtet, einen Zeitpunkt festzustellen, zu dem sich aus der Augenbewegungsmetrik 144 des Nutzers 114 eine Erkennungsschwelle des Nutzers 114 für das auf dem Bildschirm 120 dargestellte Zeichen 122 ergibt, insbesondere dass der Nutzer 114 einen Parameter des auf dem Bildschirm 120 dargestellten Zeichens 122, insbesondere die Ortsfrequenz des periodischen Musters 124, gerade noch oder gerade erst erkennen kann. Hierzu kann die Ortsfrequenz in dem periodischen Muster 124 zeitlich und/oder räumlich, insbesondere entlang der ersten Richtung 132, vorzugsweise während der Bewegung 138 des Musters, zunehmen oder abnehmen. Gleichzeitig wird die Videokamera 140 dazu verwendet, die Augenbewegungsmetrik 144 des Nutzers 114 zu ermitteln.

In einer bevorzugten Ausführung können hierzu bevorzugt Daten zur Erfassung der Augenbewegungsmetrik 144 des Nutzers 114, welche die Videokamera 140 aufgenommen und an die Auswerteeinheit 150 weitergeleitet hat, dazu verwendet werden, um die betreffende Augenbewegungsmetrik 144 des Nutzers 114 in Bezug auf das auf dem Bildschirm 120 dargestellte Zeichen 122 zu ermitteln. Im Falle einer Veränderung eines der Parameter eines für den Nutzer 114 erkennbaren Zeichens 122, insbesondere der Ortsfrequenz in dem periodischen Muster 124, wird die Augenfolgebewegung des Nutzers 114 solange der Bewegung 138 des Zeichens 122 über den Bildschirm 120 entsprechen, solange der Nutzer 114 das auf dem Bildschirm 120 dargestellte Zeichen 122 erkennen kann. Wird der Zeitpunkt erreicht, zu dem der Nutzer 114 das auf dem Bildschirm 120 dargestellte Zeichen 122, insbesondere das periodische Muster 124, gerade nicht mehr erkennen kann und das somit nicht mehr als Stimulus das Auge 112 des Nutzers 114 wirken kann, so weicht die Augenfolgebewegung des Nutzers 114 von der Bewegung 138 des Zeichens 122 auf dem Bildschirm 120 ab. Wird umgekehrt der Zeitpunkt erreicht, zu dem der Nutzer 114 das auf dem Bildschirm 120 dargestellte Zeichen 122, insbesondere das periodische Muster 124, erstmals gerade erkennen kann und das somit erstmals als Stimulus für das Auge 112 des Nutzers 114 wirken kann, so wird die Augenfolgebewegung des Nutzers 114 nun beginnen, der Bewegung 138 des Zeichens 122 auf dem Bildschirm 120 zu folgen.

In einer bevorzugten Ausführung kann die Auswerteeinheit 150 hierbei bevorzugt eine Schwelle festsetzen, mittels der ein Grad der Abweichung der Augenfolgebewegung des Nutzers 114 von der Bewegung 138 des Zeichens 122 auf dem Bildschirm 120 als der gesuchte Zeitpunkt festgelegt wird. Andere Arten der Festlegung des gesuchten Zeitpunkts sind jedoch denkbar.

Mittels der Videokamera 140 kann somit das gewünschte Bild der Augenpartie 146 des Nutzers 114 an jedem beliebigen Ort aufgenommen werden. Insbesondere mittels Bildverarbeitung, die vorzugsweise durch die Auswerteeinheit 150 durchgeführt werden kann, können aus der aufgenommenen Bildfolge geometrische Daten der Pupille 152, insbesondere eine Lage 154 und ein Durchmesser 156 der Pupille 152 im Auge 112 des Nutzers 114, ermittelt werden. Alternativ oder zusätzlich kann auch eine Bestimmung eines so genannten "Weiß-zu-weiß Abstandes" im Auge 112 des Nutzers 114, für den normierte Daten erhältlich sind.

Erfindungsgemäß ist die Auswerteeinheit 150 weiterhin dazu eingerichtet, einen Wert für den Refraktionsfehler des Auge 112 des Nutzers 114 aus einer Angebe des Zeitpunkts zu bestimmen, zu dem sich aus der Augenbewegungsmetrik 144 des Nutzers 114 die Erkennungsschwelle des Nutzers 114 für das auf dem Bildschirm 120 dargestellte Zeichen 122 ergibt. Hierzu werden die Daten zur Erfassung der Augenfolgebewegung des Nutzers 114, welche die Videokamera 140 aufgenommen hat, an die Auswerteeinheit 150 weitergeleitet, die dazu eingerichtet ist, hieraus den gewünschten Zeitpunkt festzustellen. Weiterhin sind aufgrund der der elektronischen Ansteuerung des Bildschirms 120 auf dem Smartphone 118 die Parameter des auf dem Bildschirm 120 dargestellten Zeichens 122, insbesondere die Ortsfrequenz des periodischen Musters 124, bekannt und können somit von der Auswerteeinheit 150 zur gewünschten Auswertung verwendet werden. In einer besonders bevorzugten Ausführung kann die Auswerteeinheit 150 hierzu weiterhin dazu eingerichtet sein, den gewünschten Parameter des Zeichens 122, insbesondere die Ortsfrequenz des periodischen Musters 124, durch Ansteuerung des Bildschirms 120 einzustellen.

In einer weiteren Ausführung kann zusätzlich ein als Pupillendistanz 158 bezeichneter Abstand zwischen der Videokamera 140 und dem Auge 112 des Nutzers 114 ermittelt werden. Zur Bestimmung der Pupillendistanz 158 kann eine Abstandsmessung durchgeführt werden, bevorzugt eine Abstandsmessung, über die das Smartphone 118 bereits verfügt. Alternativ oder zusätzlich kann die Pupillendistanz 158 durch Triangulation über eine bekannte Pixelanzahl der Videokamera 140 bei Detektion eines bekannten Gegenstandes oder Bildinhaltes durch die Videokamera 140 bestimmt werden. Die Pupillendistanz 158 kann insbesondere dazu verwendet werden, um einen Abstand in dem Zeichen 122, insbesondere den oben erwähnten räumlichen Abstand 130 zwischen benachbart angeordneten gleichartigen Punkten, insbesondere zwischen den benachbarten Maxima 126 oder zwischen den benachbarten Minima 128, der dem Kehrwert der Ortsfrequenz in dem periodischen Muster 124 entspricht, mit höherer Genauigkeit ermitteln zu können.

In einer besonders bevorzugten Ausführung der vorliegenden Erfindung kann aus dem zu dem Zeitpunkt festgelegten Parameter des Zeichens 122, insbesondere der Ortsfrequenz des periodischen Musters 124 zunächst eine Kontrastempfindlichkeit *ε* des Auges 112 des Nutzers 114 ermittelt werden. Wie oben bereits erwähnt, gibt die Kontrastempfindlichkeit *ε* hierbei ein Maß für einen Abstand an, den das Auge 112 des Nutzers 114 noch als unterscheidbar wahrnehmen kann, insbesondere den oben erwähnten räumlichen Abstand 130 zwischen benachbart angeordneten gleichartigen Punkten, insbesondere zwischen den benachbarten Maxima 126 oder zwischen den benachbarten Minima 128, der dem Kehrwert der Ortsfrequenz in dem periodischen Muster 124 entspricht.

Figur 2 zeigt eine schematische Darstellung, in der die gemäß dem vorliegenden Verfahren ermittelte Kontrastempfindlichkeit *ε* des Auges 110 des Nutzers 114 gegenüber der Ortsfrequenz *f* des periodischen Musters 124 aufgetragen ist. Die Kontrastempfindlichkeit *ε* des Auges 112 des Nutzers 114 kann hierbei, wie in Figur 2 schematisch mit den Symbolen "x" für jeweilige Messwerte angegeben, für verschiedene Ortsfrequenzen *f* ermittelt werden. Erfindungsgemäß wird vorgeschlagen, an die jeweiligen Messwerte eine ausgewählte Kontrastempfindlichkeitsfunktion 160, 160', 160" möglichst gut anzupassen.

Der Verlauf der Kontrastempfindlichkeitsfunktion 160, 160', 160" ist bekannt und weist einen glockenförmigen Verlauf auf, welcher üblicherweise ein Maximum bei einer Ortsfrequenz von 5 bis 6 Perioden pro Grad erreicht. Zu höheren Ortsfrequenzen *f* hin fällt die Kontrastempfindlichkeitsfunktion 160, 160', 160" steil ab, während ein ebenfalls beobachtbarer Abfall zu niedrigeren Ortsfrequenzen *f* hin weniger steil ausgeprägt nist. Ein jeweiliges Maximum 162, 162', 162" der Kontrastempfindlichkeitsfunktion 160, 160', 160"stellt das höchstmögliche Auflösungsvermögen des Auges 112 des Nutzers 116 dar. Weiterhin kann eine Fläche 164, 164', 164" unter der Kontrastempfindlichkeitsfunktion 160, 160', 160" als sichtbarer Bereich betrachtet werden, was bedeutete, dass nur solche Gegenstände von dem Nutzer 114 erkannt werden können, die einen Kontrast und eine Struktur aufweisen, die innerhalb der jeweiligen Fläche 164, 164', 164" liegen,.

Wie aus Figur 2 hervorgeht, nimmt jeder der dort dargestellten Kontrastempfindlichkeitsfunktionen 160, 160', 160" einen jeweils anderen Verlauf, wobei jeder der Kontrastempfindlichkeitsfunktionen 160, 160', 160"eine ausgewählte Defokussierung des Auges 112 als Parameter umfasst. Folglich kann genau eine der Kontrastempfindlichkeitsfunktionen 160, 160', 160" möglichst gut an die erfassten Messwerte "x" angepasst werden, da das Auge 112 genau einen Wert für die Defokussierung aufweist. Somit lässt sich aus dem Parameter der am besten passenden Kontrastempfindlichkeitsfunktionen 160' die zugehörige Defokussierung des Auges 112 des Nutzers 114 ermitteln. Aus der so ermittelten Defokussierung des Auges 112 des Nutzers 114 kann festgestellt werden, welche sphärozylindrische Ausgestaltung des Brillenglases zu einem weitgehenden Ausgleich der Refraktionsfehler des Auges 112 und damit zu einer möglichst optimalen Bildqualität für den Nutzer 114 führt. Somit kann der Wert für den Refraktionsfehler des Auges 112 des Nutzers 114 letztlich aus der derjenigen Kontrastempfindlichkeitsfunktion 160' des Auges 112 des Nutzers 114 bestimmt werden, welche am besten an die Messwerte für die Kontrastempfindlichkeit *ε* des Auges 112 des Nutzers 114 für verschiedene Ortsfrequenzen *f* angepasst werden kann. Anderer Arten der Erfassung von Messwerten und ihrer Auswertung sind jedoch denkbar.

In einer besonderen Ausführung kann die Defokussierung in mindestens zwei Meridianen bestimmt werden. Hierzu kann vorzugsweise das periodische Muster 124 gemäß Figur 1 auf dem Bildschirm 120 zunächst entlang der ersten Richtung 132 und hieran anschließend (nicht dargestellt) entlang der zweiten Richtung 134, die vorzugsweise senkrecht zu der ersten Richtung 132 angeordnet ist, auf dem Bildschirm 120 dargestellt werden. Auf diese Weise können für ein sphärozylindrisches Brillenglas mit astigmatischer Wirkung die Scheitelbrechwerte jedes der beiden Hauptschnitte, die senkrecht zueinander stehen, nacheinander ermittelt werden. Für Einzelheiten hierzu wird auf WO 2018/077690 A1 verwiesen.

Figur 3 zeigt schematisch ein Ablaufdiagramm eines bevorzugten Ausführungsbeispiels eines erfindungsgemäßen Verfahrens 210 zur Bestimmung des Refraktionsfehlers des Auges 112 des Nutzers 114.

In einem Darstellungsschritt 212 erfolgt hierzu gemäß Schritt a) die Darstellung des Zeichens 122 auf dem Bildschirm 120, wobei ein Parameter des auf dem Bildschirm 120 dargestellten Zeichens 122 verändert wird.

In einem Erfassungsschritt 214 erfolgt gemäß Schritt b) die Erfassung der Augenbewegungsmetrik 144 des Auges 112 des Nutzers 114 in Abhängigkeit von dem gemäß dem Darstellungsschritt 212 auf dem Bildschirm 120 dargestellten Zeichen 122.

In einem Feststellungsschritt 216 erfolgt gemäß Schritt c) die Feststellung des Zeitpunkts, zu dem sich aus der Augenbewegungsmetrik des Auges 112 des Nutzers 114 in dem Erfassungsschritt 214 eine Erkennungsschwelle des Nutzers 114 für das auf dem Bildschirm 120 dargestellte Zeichen 122 ergibt, so dass der Nutzer 114 das gemäß dem Darstellungsschritt 212 auf dem Bildschirm 120 dargestellte Zeichen 122 gerade noch oder gerade erst erkennen kann.

In einem Bestimmungsschritt 218 erfolgt gemäß Schritt d) die Bestimmung eines Wertes 220 für den Refraktionsfehler des Auges 112 des Nutzers 114 aus dem Parameter, welcher zu dem Zeitpunkt, der in dem Feststellungsschritt 216 ermittelt wurde, zur Darstellung des Zeichens 122 auf dem Bildschirm 120 in dem Darstellungsschritt 212 festgelegt war.

### Bezugszeichenliste

- 110: Vorrichtung
- 112: Auge
- 114: Nutzer
- 116: mobiles Kommunikationsgerät
- 118: Smartphone
- 120: Bildschirm
- 122: Zeichen
- 124: Muster
- 126: Maximum
- 128: Minimum
- 130: räumlicher Abstand
- 132: erste Richtung
- 134: zweite Richtung
- 136: Streifen
- 138: Bewegung
- 140: Videokamera
- 142: Frontkamera
- 144: Augenbewegungsmetrik
- 146: Augenpartie
- 148: Gehäuse
- 150: Auswerteeinheit
- 152: Pupille
- 154: Lage der Pupille
- 156: Pupillendurchmesser
- 158: Abstand (Pupillendistanz)
- 160, 160'...: Kontrastempfindlichkeitsfunktion
- 162, 162'...: Maxima
- 164, 164'...: Fläche
- 210: Verfahren zur Bestimmung eines Refraktionsfehlers eines Auges eines Nutzers
- 212: Darstellungsschritt
- 214: Erfassungsschritt
- 216: Feststellungsschritt
- 218: Bestimmungsschritt
- 220: Wert für den Refraktionsfehler des Auges des Nutzers

## Patentansprüche

1. Verfahren (210) zur Bestimmung eines Refraktionsfehlers eines Auges (112) eines Nutzers (114), wobei das Verfahren (210) die folgenden Schritte umfasst:
a) Darstellen eines Zeichens (122) auf einem Bildschirm (120), wobei ein Parameter des auf dem Bildschirm (120) dargestellten Zeichens (122) verändert wird;
b) Erfassen einer Augenbewegungsmetrik (144) des Auges (112) des Nutzers (114) in Abhängigkeit von dem auf dem Bildschirm (120) dargestellten Zeichen (122); und
c) Feststellen eines Zeitpunkts, zu dem sich aus der Augenbewegungsmetrik (144) des Auges (112) des Nutzers (114) eine Erkennungsschwelle des Nutzers (114) für das auf dem Bildschirm (120) dargestellte Zeichen (122) ergibt;
**gekennzeichnet durch**
d) Bestimmen eines Wertes (220) für den Refraktionsfehler des Auges (112) des Nutzers (114) aus dem zu dem Zeitpunkt festgelegten Parameter.

2. Verfahren (210) nach dem vorangehenden Anspruch, **dadurch gekennzeichnet, dass** der Parameter des auf dem Bildschirm (120) dargestellten Zeichens (122) verändert wird, während das auf dem Bildschirm (120) dargestellte Zeichen (122) eine Bewegung (138) über den Bildschirm (120) ausführt.

3. Verfahren (210) nach dem vorangehenden Anspruch, **dadurch gekennzeichnet, dass** die Bewegung (138) des auf dem Bildschirm (120) dargestellten Zeichens (122) kontinuierlich oder in Sprüngen erfolgt.

4. Verfahren (210) nach einem der beiden vorangehenden Ansprüche, **dadurch gekennzeichnet, dass** der Zeitpunkt dadurch festgestellt wird, dass die Augenbewegungsmetrik (144) des Nutzers (114) der Bewegung (138) des auf dem Bildschirm (120) dargestellten Zeichens (122) gerade noch folgt oder gerade erst folgt.

5. Verfahren (210) nach einem der vorangehenden Ansprüche, **dadurch gekennzeichnet, dass** aus dem zu dem Zeitpunkt festgelegten Parameter zunächst eine Kontrastempfindlichkeit des Auges (112) des Nutzers (114) ermittelt wird, und dass hieran anschließend der Wert (220) für den Refraktionsfehler des Auges (112) des Nutzers (114) aus der Kontrastempfindlichkeit des Auges (112) des Nutzers (114) bestimmt wird.

6. Verfahren (210) nach dem vorangehenden Anspruch, **dadurch gekennzeichnet, dass** zunächst eine Kontrastempfindlichkeitsfunktion (160') festgestellt wird, welche an ermittelte Werte der Kontrastempfindlichkeit des Auges (112) des Nutzers (114) angepasst ist, dann aus der festgestellten Kontrastempfindlichkeitsfunktion (160') eine Defokussierung des Auges (112) des Nutzers (114) ermittelt wird, und schließlich aus der Defokussierung des Auges (112) des Nutzers (114) der Wert (220) für den Refraktionsfehler des Auges (112) des Nutzers (114) bestimmt wird.

7. Verfahren (210) nach einem der vorangehenden Ansprüche, **dadurch gekennzeichnet, dass** das Zeichen (122) ein periodisches Muster (124) ist oder umfasst, wobei der Parameter des auf dem Bildschirm (120) dargestellten Musters (124) mindestens eine Ortsfrequenz ist oder umfasst.

8. Verfahren (210) nach einem der vorangehenden Ansprüche, **dadurch gekennzeichnet, dass** das Zeichen (122) zunächst in einer ersten Richtung (132) in Bezug auf eine Orientierung des Bildschirms (120) dargestellt wird und anschließend in einer gegen die erste Richtung (132) veränderten zweiten Richtung (134) dargestellt wird.

9. Verfahren (210) nach einem der vorangehenden Ansprüche, **dadurch gekennzeichnet, dass** zusätzlich ein Abstand (158) des Auges (112) des Nutzers (114) von dem Bildschirm (120) erfasst wird.

10. Verfahren (210) nach einem der vorangehenden Ansprüche, **dadurch gekennzeichnet, dass** das Verfahren (210) ausgeführt wird, während der Nutzer (114) eine Brille trägt.

11. Computerprogramm zur Bestimmung eines Refraktionsfehlers eines Auges (112) eines Nutzers (114), wobei das Computerprogramm dazu eingerichtet ist, die folgenden Schritte durchzuführen:
a) Darstellen eines Zeichens (122) auf einem Bildschirm (120), wobei ein Parameter des auf dem Bildschirm (120) dargestellten Zeichens (122) verändert wird;
b) Erfassen einer Augenbewegungsmetrik (144) des Auges (112) des Nutzers (114) in Abhängigkeit von dem auf dem Bildschirm (120) dargestellten Zeichen (122); und
c) Feststellen eines Zeitpunkts, zu dem sich aus der Augenbewegungsmetrik (144) des Auges (112) des Nutzers (114) eine Erkennungsschwelle des Nutzers (114) für das auf dem Bildschirm (120) dargestellte Zeichen (122) ergibt;
**gekennzeichnet durch**
d) Bestimmen eines Wertes (220) für den Refraktionsfehler des Auges (112) des Nutzers (114) aus dem zu dem Zeitpunkt festgelegten Parameter.

12. Verfahren zur Herstellung eines Brillenglases für ein Auge (112) eines Nutzers (114), wobei die Herstellung des Brillenglases durch Bearbeitung eines Brillenglasrohlings erfolgt, wobei der Brillenglasrohling anhand von Zentrierdaten bearbeitet wird, wobei die Zentrierdaten Anweisungen zum Ausgleich eines Refraktionsfehlers des Auges (112) des Nutzers (114) umfassen, wobei eine Bestimmung des Refraktionsfehlers des Auges (112) des Nutzers (114) die folgenden Schritte umfasst:
a) Darstellen eines Zeichens (122) auf einem Bildschirm (120), wobei ein Parameter des auf dem Bildschirm (120) dargestellten Zeichens (122) verändert wird;
b) Erfassen einer Augenbewegungsmetrik (144) des Auges (112) des Nutzers (114) in Abhängigkeit von dem auf dem Bildschirm (120) dargestellten Zeichen (122); und
c) Feststellen eines Zeitpunkts, zu dem sich aus der Augenbewegungsmetrik (144) des Auges (112) des Nutzers (114) eine Erkennungsschwelle des Nutzers (114) für das auf dem Bildschirm (120) dargestellte Zeichen (122) ergibt;
**gekennzeichnet durch**
d) Bestimmen eines Wertes (220) für den Refraktionsfehler des Auges (112) des Nutzers (114) aus dem zu dem Zeitpunkt festgelegten Parameter.

13. Vorrichtung (110) zur Bestimmung eines Refraktionsfehlers eines Auges (112) eines Nutzers (114), wobei die Vorrichtung umfasst:
- einen Bildschirm (120), der zur Darstellung eines Zeichen (122) und einer Veränderung eines Parameters des Zeichens (122) eingerichtet ist;
- mindestens eine Videokamera (140), die zur Erfassung einer Augenbewegungsmetrik (144) des Auges (112) des Nutzers (114) in Abhängigkeit von dem auf dem Bildschirm (120) dargestellten Zeichen (122) eingerichtet ist; und
- eine Auswerteeinheit (150), die zur Feststellung eines Zeitpunkts, zu dem sich aus der Augenbewegungsmetrik (144) des Auges (112) des Nutzers (114) eine Erkennungsschwelle des Nutzers (114) für das auf dem Bildschirm (120) dargestellten Zeichen (122) ergibt,
**dadurch gekennzeichnet,**
**dass** die Auswerteeinheit (150) weiterhin zur Bestimmung eines Wertes (220) für den Refraktionsfehler des Auges (112) des Nutzers (114) aus dem zu dem Zeitpunkt festgelegten Parameter eingerichtet ist.

14. Vorrichtung (110) nach dem vorangehenden Anspruch, **dadurch gekennzeichnet, dass** die Vorrichtung (110) weiterhin eine Einrichtung zur Erfassung eines Abstands (158) des Auges (112) des Nutzers (114) von dem Bildschirm (120) aufweist.

15. Vorrichtung (110) nach einer der beiden vorangehenden Ansprüche, **dadurch gekennzeichnet, dass** die Vorrichtung (110) weiterhin eine Lichtquelle (160), welche im infraroten Spektralbereich abstrahlt, umfasst, und dass die Videokamera (140) infrarotsensitiv ist.
